(19) Europäisches Patentamt
European Patent Office
Office européen des brevets

(11) EP 1 666 064 A1

(12) EUROPEAN PATENT APPLICATION
published in accordance with Art. 158(3) EPC

(43) Date of publication:
**07.06.2006 Bulletin 2006/23**

(21) Application number: **04772374.7**

(22) Date of filing: **23.08.2004**

(51) Int Cl.:
***A61K 45/00*** (1985.01) ***A61K 31/365*** (1985.01)
***A61P 35/02*** (2000.01)

(86) International application number:
**PCT/JP2004/012418**

(87) International publication number:
**WO 2005/018674 (03.03.2005 Gazette 2005/09)**

(84) Designated Contracting States:
**AT BE BG CH CY CZ DE DK EE ES FI FR GB GR HU IE IT LI LU MC NL PL PT RO SE SI SK TR**

(30) Priority: **22.08.2003 JP 2003208332**

(71) Applicant: **KYOWA HAKKO KOGYO CO., LTD.**
**Chiyoda-ku,**
**Tokyo 100-8185 (JP)**

(72) Inventors:
- **SHIOTSU, Yukimasa,**
**c/o Kyowa Hakko Kogyo Co.,Ltd.,**
**Shizuoka 411-8731 (JP)**
- **SOGA, Shiro**
**Rockville, Maryland 20852 (US)**
- **KANDA, Yutaka,**
**c/o Kyowa Kakko Kogyo Co., Ltd.,**
**Shizuoka 411-8731 (JP)**
- **AKINAGA, Shiro,**
**c/o Kyowa Kakko Kogyo Co., Ltd.,**
**Tokyo 100-8185 (JP)**

(74) Representative: **Casalonga, Axel**
**Bureau Casalonga & Josse**
**Bayerstrasse 71/73**
**80335 München (DE)**

# (54) REMEDY FOR DISEASES ASSOCIATED WITH IMMUNOGLOBULIN GENE TRANSLOCATION

(57) A medicament comprising a compound having an inhibitory action on Hsp90 or a pharmaceutically acceptable salt thereof as an active ingredient is used as a therapeutic agent for diseases associated with immunoglobulin gene translocations. In diseases associated with immunoglobulin gene translocations, abnormal enhancement of the expression of a partner gene in immunoglobulin gene translocation participates in the development of the diseases and the progress of the symptoms. By administering the compound having an inhibitory action on Hsp90 or a pharmaceutically acceptable salt thereof, degradation of protein encoded by the partner gene is promoted and the diseases can be treated.

EP 1 666 064 A1

## Description

### Technical Field

The present invention relates to a therapeutic agent for diseases associated with immunoglobulin gene translocations, a growth inhibitor of cells with immunoglobulin gene translocations, and an agent for promoting degradation of proteins encoded by partner genes in immunoglobulin gene translocations.

### Background Art

Heat shock proteins (hereinafter abbreviated as "Hsp") are a series of proteins expressed within cells when the cells are exposed to a stress environment such as heat shock, etc., and are classified into families such as Hsp90, Hsp70 and Hsp60 according to their molecular weight. These proteins are also called molecular chaperones, and generally, folding, membrane transport, association and suppression of agglutination of proteins, etc. are considered as their main functions. Hsp90 is a family of Hsps, which consists of Hsps having a molecular weight of about 90kDa. As Hsps belonging to the Hsp90 family of eukaryotes, Hsp90α, Hsp90β, Grp94, Hsp75/TRAP1, etc. have been identified. Hsps belonging to the Hsp90 family are hereinafter generically called Hsp90.

In recent years, it has been clarified that Hsp90 forms a complex specifically with a molecule involved in cell growth and tumorigenesis to participate in the cell cycle and cell growth signals. Proteins forming a complex specifically with Hsp90 are called Hsp90 client proteins. In order to maintain the function and stability of the Hsp90 client proteins within the cell, binding to Hsp90 is considered to be necessary. Examples of known Hsp90 client proteins are steroid hormone receptors (e.g., estrogen receptor, progesterone receptor and glucocorticoid receptor), non-receptor type tyrosine kinases (e.g., Src and Lck), receptor type tyrosine kinases (e.g., EGF receptor and ErbB2), serine-threonine kinases (e.g., Raf-1, cyclin-dependent kinase (Cdk) 4 and Cdk6) and fusion proteins derived from translocation of genes (e.g., Bcr-Abl and NPM-ALK). It is known that by controlling the functions of Hsp90, it is possible to change intracellular signal transduction in which these molecules that specifically bind to Hsp90 participate [Pharmacol. Ther., 79, 129-168 (1997); Biochem. Pharmacol., 56, 675-682 (1998); Invest. New Drugs, 17, 361-373 (1999)].

It has been clarified that radicicol and its derivatives bind to the ATP/ADP binding site located at the N-terminus of Hsp90 and inhibit its functions [Cell Stress Chaperones, 3, 100-108 (1998); J. Med. Chem., 42, 260-266 (1999)]. Also, ansamycin compounds such as geldanamycin and herbimycin A and their derivatives [Cell, 89, 239-250 (1997); J. Natl. Cancer Inst., 92, 242-248 (2000)], and purine derivatives (WO02/36075) have been reported to be compounds that bind to the same site of the N-terminus of Hsp90. Hsp90 functions by forming a molecular complex with the above-mentioned Hsp90 client proteins together with coupling molecules such as p50/Cdc37 and p23. These low molecular weight compounds are considered to show various biological activities including suppression of growth of cancer cells and induction of apoptosis by binding to the ATP/ADP binding site at the N-terminus of Hsp90, thereby changing the construction of the molecular complexes containing Hsp90, and consequently altering the function, intracellular localization or intracellular stability of Hsp90 client proteins [Invest. New Drugs, 17, 361-373 (1999)]. Coumarin compounds such as novobiocin are reported to show effects similar to those of the above-mentioned low molecular weight compounds which bind to the N-terminus, by binding to the C-terminal site (contained in amino acids 380-728) of Hsp90 [J. Natl. Cancer Inst., 92, 242-248 (2000)]. Geldanamycin derivatives [Invest. New Drugs, 17, 361-373 (1999)] and radicicol derivatives [Cancer Res., 59, 2931-2938 (1999); Blood, 96, 2284-2291 (2000); Cancer Chemother. Pharmacol., 48, 435-445 (2001)] are reported to show antitumor effects in animal models as well.

As the stability of fusion proteins derived from gene translocations such as Bcr-Abl and NPM-ALK is considered to decrease due to a compound having an inhibitory action on Hsp90, it has been proposed to treat diseases associated with gene translocations such as leukemia and solid tumor with compounds having an inhibitory action on Hsp90 (WO02/069900).

It is reported that B-cell (including plasmacyte) tumors are often associated with immunoglobulin gene translocations on the q32 region of chromosome 14 (hereinafter indicated as 14q32). In the case of multiple myeloma, for example, immunoglobulin gene translocations on 14q32 are more frequently observed as the stage of the disease progresses. When immunoglobulin gene translocations on 14q32 of tumor cells collected from patients are examined, the translocations are reported to exist in 50% of the patients with monoclonal gammopathy which is considered to be a premyeloma disease, in 60-65% of intramedullary myeloma patients, in 70-80% of extramedullary myeloma patients, and in more than 90% of established myeloma cell lines [Oncogene, 20, 5611-5622 (2001)].

Examples of chromosomal regions of partners that bring about translocation with immunoglobulin genes on 14q32 and those of genes which become a translocation partner (partner genes) on the regions are mainly cyclin D1 gene on 11q13, cyclin D3 gene on 6p21, FGFR3 (fibroblast growth factor receptor 3) gene and MMSET (multiple myeloma SET domain) gene on 4p16 and c-maf gene on 16q23 [Oncogene, 20, 5611-5622 (2001)]. Also, MUM1/IRF-4 (multiple myeloma cancer gene 1/interferon-regulatory factor 4) gene on 6p25, IRTA1 (immunoglobulin super family receptor

translocation-associated gene 1) gene and IRTA2 gene on 1q21-24, mafB gene on 20q12, etc. have so far been reported to become a partner gene for immunoglobulin gene translocations on 14q32 [Oncogene, 20, 5611-5622 (2001)]. It is considered that on abnormal chromosomes resulting from immunoglobulin gene translocations on 14q32, proteins (cyclin D1, FGFR3, MMSET, c-maf, cyclin D3, IRF-4, IRTA1, IRTA2, mafB, etc.) encoded by the translocation partner genes undergo abnormally elevated expression under an enhancer of immunoglobulin genes on chromosome 14 to participate in malignant transformation of cells [Oncogene, 20, 5611-5622 (2001)]. Further, 1q10-12, 2p23, 3q21, 4q22-33, 9q13, 11q23, 20q12, 21q22, 22q12, etc. are reported as the chromosomal regions known to be the partners for immunoglobulin gene translocations on 14q32 although proteins encoded are not identified, and abnormal gene expression resulting from translocation between these chromosomal regions and immunoglobulin genes on 14q32 may possibly participate in tumorigenesis [Oncogene, 20, 5611-5622 (2001)].

However, in conventional treatments for cancers associated with immunoglobulin gene translocations on 14q32, ordinary chemotherapeutic agents are used, but specific treatments targeted to molecules encoded by translocation partner genes of which abnormal expression is induced as a result of immunoglobulin gene translocation have not been carried out. In the case of multiple myeloma, for example, DNA-alkylating agents [e.g., melphalan, cyclophosphamyde and N,N'-bis(2-chloroethyl)-N-nitrosourea (BCNU)], topoisomerase inhibitors (e.g., doxorubicin and etoposide), glucocorticoids (e.g., predonisone and dexamethasone), tubulin inhibitors (e.g., vincristine) and the like are generally used alone or in combination according to the purpose of treatment. Although antitumor effects of these chemotherapeutic agents are observed at the initial stage of treatment, with the 5-year survival rate of 28% and 10-year survival rate of 3% due to recurrence and drug-resistance caused later, no sufficient medicinal effect is obtained only with conventional therapeutic agents [Oncogene, 20, 5611-5622 (2001); Hematology (Am. Soc. Hematol. Educ. Program), 157-177 (2001)] .

There have been reports that geldanamycin, a compound having an inhibitory action on Hsp90, inhibits growth of various multiple myeloma cell lines and multiple myeloma cells obtained from patients [Blood, 98, 1587 (2001)] and that administration of 17-allylamino-17-demethoxy geldanamycin, a geldanamycin derivative, extended survival time of mice to which multiple myeloma cells were transplanted [Blood, 100, 392 (2002)]. However, these reports do not refer to the relation between Hsp90 and immunoglobulin gene translocations and do not suggest that a compound having an inhibitory action on Hsp90 targets proteins encoded by partner genes in immunoglobulin gene translocations.

Fusion proteins derived from gene translocations such as Bcr-Abl and NPM-ALK are known to be Hsp90 client proteins, but it is not clear whether proteins encoded by abnormal genes induced as a result of the translocations are all Hsp90 client proteins or not. Also, it has not so far been clarified whether or not proteins encoded by the translocation partner genes of which abnormal expression is induced as a result of immunoglobulin gene translocations on 14q32 are Hsp90 client proteins and whether or not their functions and intracellular stability are dependent on binding to Hsp90. Further, there is no report that a compound having an inhibitory action on Hsp90 shows an antitumor effect on cancer cells with immunoglobulin gene translocations.

## Disclosure of the Invention

An object of the present invention is to provide a therapeutic agent for diseases associated with immunoglobulin gene translocations, such as multiple myeloma, based on new mechanisms. The present invention relates to inventions of the following (1) to (16).

(1) A therapeutic agent for a disease associated with immunoglobulin gene translocations comprising, as an active ingredient, a compound having an inhibitory action on Hsp90 or a pharmaceutically acceptable salt thereof.
(2) The therapeutic agent according to (1), wherein the compound having an inhibitory action on Hsp90 has an inhibitory action on at least one Hsp90 among Hsp90α, Hsp90β, Grp94 and Hsp75/TRAP1.
(3) The therapeutic agent according to (1), wherein the compound having an inhibitory action on Hsp90 is a compound binding to the ATP/ADP binding site of Hsp90.
(4) The therapeutic agent according to (3), wherein the compound binding to the ATP/ADP binding site of Hsp90 is radicicol or a derivative thereof represented by the following general formula (I):

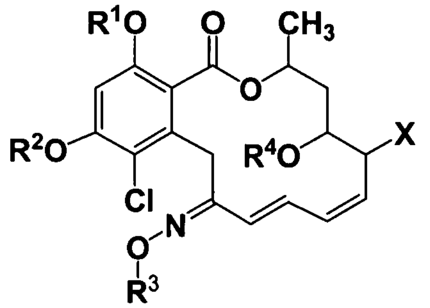

(I)

[wherein $R^1$ and $R^2$, which may be the same or different, each represent a hydrogen atom, alkanoyl, alkenoyl, tert-butyldiphenylsilyl or tert-butyldimethylsilyl,

$R^3$ represents Y-$R^5$ {wherein Y represents substituted or unsubstituted alkylene, and $R^5$ represents CON$R^6R^7$ [wherein $R^6$ represents a hydrogen atom, hydroxy, substituted or unsubstituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or N$R^8R^9$ (wherein $R^8$ and $R^9$, which may be the same or different, each represent a hydrogen atom, substituted or unsubstituted lower alkyl, substituted or unsubstituted higher alkyl, substituted or unsubstituted lower cycloalkyl, substituted or unsubstituted aryl, a substituted or unsubstituted heterocyclic group, substituted or unsubstituted alkanoyl, substituted or unsubstituted aroyl, carbonyl bonded to a substituted or unsubstituted heterocyclic group, or substituted or unsubstituted arylcarbamoyl), and $R^7$ represents hydroxy, substituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or N$R^{10}R^{11}$ (wherein $R^{10}$ and $R^{11}$ have the same significances as the above $R^8$ and $R^9$, respectively), or $R^6$ and $R^7$ form a substituted or unsubstituted heterocyclic group together with the adjacent nitrogen atom], $CO_2R^{12}$ (wherein $R^{12}$ represents substituted lower alkyl, substituted or unsubstituted higher alkyl, substituted or unsubstituted lower cycloalkyl, substituted or unsubstituted alkenyl, substituted or unsubstituted aryl, or a substituted or unsubstituted heterocyclic group), substituted or unsubstituted aryl, substituted or unsubstituted pyridyl, substituted or unsubstituted pyridonyl, substituted or unsubstituted pyrrolidonyl, substituted or unsubstituted uracilyl, substituted or unsubstituted piperidyl, substituted or unsubstituted piperidino, substituted or unsubstituted pyrrolidinyl, substituted or unsubstituted morpholino, substituted or unsubstituted morpholinyl, substituted or unsubstituted piperazinyl, substituted or unsubstituted thiomorpholino, or substituted or unsubstituted dioxolanyl}, CO$R^{13}$ [wherein $R^{13}$ represents a hydrogen atom, substituted or unsubstituted lower alkyl, substituted or unsubstituted higher alkyl, substituted or unsubstituted aryl, substituted or unsubstituted lower alkoxy, or N$R^{14}R^{15}$ (wherein $R^{14}$ and $R^{15}$, which may be the same or different, each represent a hydrogen atom, substituted or unsubstituted lower alkyl, substituted or unsubstituted higher alkyl, substituted or unsubstituted aryl, or substituted or unsubstituted pyridyl, or $R^{14}$ and $R^{15}$ form a substituted or unsubstituted heterocyclic group together with the adjacent nitrogen atom)] or substituted or unsubstituted aryl,

X represents halogen, and $R^4$ represents a hydrogen atom, alkanoyl, alkenoyl or -SO-Z {wherein Z represents formula (A):

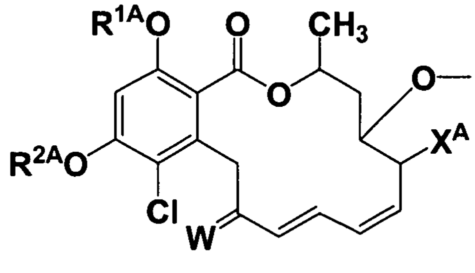

(A)

[wherein $R^{1A}$ and $R^{2A}$ have the same significances as the above $R^1$ and $R^2$, respectively, $X^A$ represents halogen, and W represents O or N-O-$R^{3A}$ (wherein $R^{3A}$ has the same significance as the above $R^3$)]}, or X and $R^4$ together represent a single bond].

(5) The therapeutic agent according to any one of (1) to (4), wherein the immunoglobulin gene translocation is at

the q32 region on chromosome 14 (14q32).

(6) The therapeutic agent according to (5), wherein the translocation at 14q32 is t(11;14)(q13;q32), t(6;14)(p21; q32), t(4;14)(p16;q32), t(14:16)(q32;q23), t(6;14)(p25;q32), t(1;14)(q21-24;q32), t(14;20)(q32;q12), t(1;14)(q10-12; q32), t(2;14)(p13;q32), t(3;14)(q21;q32), t(4;14)(q22-33;q32), t(9;14)(p13;q32), t(11;14)(q23;q32), t(12;14)(p13; q32), t (14;21) (q32;q22) or t(14:22)(q32;q12).

(7) The therapeutic agent according to (5), wherein the partner gene in the translocation at 14q32 is cyclin D1 gene on 11q13, cyclin D3 gene on 6p21, FGFR3 gene or MMSET gene on 4p16, c-maf gene on 16q23, MUM1/IRF-4 gene on 6p25, IRTA1 gene or IRTA2 gene on 1q21-24, or mafB gene on 20q12.

(8) The therapeutic agent according to any one of (1) to (7), wherein the disease associated with immunoglobulin gene translocations is a hematopoietic tumor.

(9) The therapeutic agent according to (8), wherein the hematopoietic tumor is leukemia, multiple myeloma or lymphoma.

(10) A growth inhibitor for a cell with immunoglobulin gene translocation comprising, as an active ingredient, a compound having an inhibitory action on Hsp90 or a pharmaceutically acceptable salt thereof.

(11) An agent for promoting degradation of a protein encoded by a partner gene in immunoglobulin gene translocation comprising, as an active ingredient, a compound having an inhibitory action on Hsp90 or a pharmaceutically acceptable salt thereof.

(12) Use of a compound having an inhibitory action on Hsp90 or a pharmaceutically acceptable salt thereof for the manufacture of a pharmaceutical composition for the treatment of a disease associated with immunoglobulin gene translocations.

(13) A method of screening for a patient, which comprises:

collecting cells from patients; examining the presence or absence of immunoglobulin gene translocation on the chromosome of the cells; and
selecting a patient with immunoglobulin gene translocation as a target patient to administer a medicament comprising, as an active ingredient, a compound having an inhibitory action on Hsp90 or a pharmaceutically acceptable salt thereof as a therapeutic agent.

(14) The method according to (13), wherein the patient is a hematopoietic tumor patient.

(15) The method according to (14), wherein the hematopoietic tumor is leukemia, multiple myeloma or lymphoma.

(16) A method of treating a disease associated with immunoglobulin gene translocations, which comprises:

collecting cells from patients; examining the presence or absence of immunoglobulin gene translocation on the chromosome of the cells; and
administering a medicament comprising, as an active ingredient, a compound having an inhibitory action on Hsp90 or a pharmaceutically acceptable salt thereof to a patient with immunoglobulin gene translocation.

The present inventors have found that, in cells with immunoglobulin gene translocation, compounds having an inhibitory action on Hsp90 promote intracellular degradation of a protein encoded by translocation partner gene of which expression is abnormally enhanced as a result of the translocation and suppress growth of the cells. Accordingly, a medicine comprising a compound having an inhibitory action on Hsp90 or a pharmaceutically acceptable salt thereof as an active ingredient can be used as a therapeutic agent for diseases associated with immunoglobulin gene translocations, such as multiple myeloma.

"Translocation" means genetic abnormality in which a part of a chromosome migrates to another site of the same chromosome or another chromosome. "Immunoglobulin gene translocation" means genetic abnormality in which a chromosomal region comprising a part or whole of the immunoglobulin genes on chromosome 14 migrates to a chromosome other than chromosome 14 or another site of chromosome 14. "Immunoglobulin gene" includes not only the regions to be transcribed including introns but also transcription-controlling regions such as promoters and enhancers. "Translocation at 14q32" means genetic abnormality in which a fragment of chromosome 14 cleaved in the 14q32 region migrates to another chromosome. Further, "Partner gene" means, in translocation in which two chromosomes undergo cleavage, mutually exchange the partner for fusion and fuse with a chromosomal fragment of the partner, genes present at the breakpoint of the partner chromosome or in its vicinity. A translocation in which "x" chromosome which underwent cleavage at "a" region and "y" chromosome which underwent cleavage at "b" region mutually exchange the partner and fuse together with a chromosomal fragment of the other is expressed as t(x;y)(a;b). In this case, the region where the chromosomal breakpoint of the partner exists is called "chromosomal region of the translocation partner". The present invention is explained below.

1. Diseases associated with immunoglobulin gene translocations

Diseases associated with immunoglobulin gene translocations refer to diseases in which immunoglobulin gene translocations are present in the chromosome of the cells at the diseased site of patients.

Medicaments comprising a compound having an inhibitory action on Hsp90 or a pharmaceutically acceptable salt thereof as an active ingredient may be used as a therapeutic agent for any diseases associated with immunoglobulin gene translocations.

Examples of diseases associated with immunoglobulin gene translocations include hematopoietic tumors, particularly, leukemia, multiple myeloma and lymphoma. Multiple myeloma, in particular, involves immunoglobulin gene translocations at 14q32 with a high probability, so medicaments comprising a compound having an inhibitory action on Hsp90 or a pharmaceutically acceptable salt thereof as an active ingredient can be used as a therapeutic agent therefor.

Immunoglobulin gene translocation in diseases may be any translocation insofar as it is immunoglobulin gene translocation and particularly includes translocation at the 14q32 chromosomal region where immunoglobulin H chain gene is present. Examples of translocations at 14q32 are those indicated as t (11;14) (q13;q32), t(6;14) (p21;q32), t (4; 14) (p16;q32), t (14; 16) (q32;q23), t (6; 14) (p25;q32), t(1; 14) (q21-24;q32), t(14; 20) (q32;q12), t(1; 14) (q10-12;q32), t (2;14) (p13;q32), t(3; 14) (q21;q32), t (4;14) (q22-33;q32), t (9;14) (p13;q32), t(11; 14) (q23;q32), t(12;14) (p13;q32), t (14;21)(q32;q22) and t (14;22) (q32;q12) in which one of 11q13, 6p21, 4p16, 16q23, 6p25, 1q21-24, 20q121q10-12, 2p23, 3q21, 4q22-33, 9q13, 11q23, 20q12, 21q22 and 22q12 is the chromosomal region of the translocation partner at 14q32. Further, these translocations at 14q32 include those in which the partner gene is cyclin D1 gene on 11q13, cyclin D3 gene on 6p21, FGFR3 gene and MMSET gene on 4p16, c-maf gene on 16q23, MUM1/IRF-4 gene on 6p25, IRTA1 gene and IRTA2 gene on 1q21-24 or mafB gene on 20q12. Examples of proteins that are considered to undergo abnormal enhancement of expression as a result of immunoglobulin gene translocations are cyclin D1, cyclin D3, FGFR3, MMSET, c-maf, MUM1/IRF-4, IRTA1, IRTA2 and mafB which are encoded by the translocation partner genes.

2. Compounds having an inhibitory action on Hsp90

The compounds having an inhibitory action on Hsp90 used in the present invention may be substances of any structure insofar as they inhibit functions of Hsp90, for example, a function of forming a molecular complex with an Hsp90 client protein. The compounds having an inhibitory action on Hsp90 used in the present invention include those that inhibit functions of at least one of Hsp90α, Hsp90β, Grp94 and Hsp75/TRAP1 among Hsps belonging to Hsp90.

Examples of the compounds having an inhibitory action on Hsp90 include those that bind to the ATP/ADP binding site located at the N-terminus of Hsp90 and inhibit its functions, and those that inhibit the functions of Hsp90 by binding to the C-terminal region of Hsp90 (corresponding to amino acids 380-728). Examples of the compounds that bind to the ATP/ADP binding site of Hsp90 and inhibit its functions include radicicol and its derivatives, ansamycin compounds such as geldanamycin and herbimycin A and their derivatives, and purine derivatives binding to Hsp90 described in WO02/36075. Examples of medicaments that inhibit the functions of Hsp90 by binding to the C-terminal region of Hsp90 include coumarin compounds such as novobiocin, chlorobiocin and coumermycin Al and their derivatives. Radicicol, geldanamycin, herbimycin A and novobiocin are commercially available from Carbiochem. They can also be purified and isolated from the culture broth of a microorganism by the methods described in US3595955, J. Biol. Chem., 273, 822 (1998), etc.

Examples of the radicicol derivatives are KF58333 [Blood, 96, 2284 (2000)], KF25706 [Cancer Res., 59, 2931 (1999)], the derivatives described in Japanese Published Unexamined Patent Application No. 226991/1992, in which the phenolic hydroxyl group is modified with various acyl groups, the oxime derivatives of dienone described in WO96/33989 or WO99/55689, and radicicol derivatives represented by general formula (I):

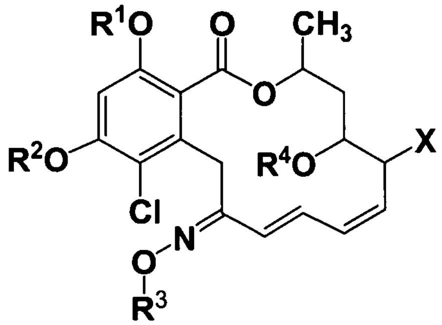

(I)

[wherein $R^1$ and $R^2$, which may be the same or different, each represent a hydrogen atom, alkanoyl, alkenoyl, tert-butyldiphenylsilyl or tert-butyldimethylsilyl,

$R^3$ represents Y-$R^5$ {wherein Y represents substituted or unsubstituted alkylene, and $R^5$ represents $CONR^6R^7$ [wherein $R^6$ represents a hydrogen atom, hydroxy, substituted or unsubstituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or $NR^8R^9$ (wherein $R^8$ and $R^9$, which may be the same or different, each represent a hydrogen atom, substituted or unsubstituted lower alkyl, substituted or unsubstituted higher alkyl, substituted or unsubstituted lower cycloalkyl, substituted or unsubstituted aryl, a substituted or unsubstituted heterocyclic group, substituted or unsubstituted alkanoyl, substituted or unsubstituted aroyl, carbonyl bonded to a substituted or unsubstituted heterocyclic group, or substituted or unsubstituted arylcarbamoyl), and $R^7$ represents hydroxy, substituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or $NR^{10}R^{11}$ (wherein $R^{10}$ and $R^{11}$ have the same significances as the above $R^8$ and $R^9$, respectively), or $R^6$ and $R^7$ form a substituted or unsubstituted heterocyclic group together with the adjacent nitrogen atom], $CO_2R^{12}$ (wherein $R^{12}$ represents substituted lower alkyl, substituted or unsubstituted higher alkyl, substituted or unsubstituted lower cycloalkyl, substituted or unsubstituted alkenyl, substituted or unsubstituted aryl, or a substituted or unsubstituted heterocyclic group), substituted or unsubstituted aryl, substituted or unsubstituted pyridyl, substituted or unsubstituted pyridonyl, substituted or unsubstituted pyrrolidonyl, substituted or unsubstituted uracilyl, substituted or unsubstituted piperidyl, substituted or unsubstituted piperidino, substituted or unsubstituted pyrrolidinyl, substituted or unsubstituted morpholino, substituted or unsubstituted morpholinyl, substituted or unsubstituted piperazinyl, substituted or unsubstituted thiomorpholino, or substituted or unsubstituted dioxolanyl}, $COR^{13}$ [wherein $R^{13}$ represents a hydrogen atom, substituted or unsubstituted lower alkyl, substituted or unsubstituted higher alkyl, substituted or unsubstituted aryl, substituted or unsubstituted lower alkoxy, or $NR^{14}R^{15}$ (wherein $R^{14}$ and $R^{15}$, which may be the same or different, each represent a hydrogen atom, substituted or unsubstituted lower alkyl, substituted or unsubstituted higher alkyl, substituted or unsubstituted aryl, or substituted or unsubstituted pyridyl, or $R^{14}$ and $R^{15}$ form a substituted or unsubstituted heterocyclic group together with the adjacent nitrogen atom)] or substituted or unsubstituted aryl,

X represents halogen, and $R^4$ represents a hydrogen atom, alkanoyl, alkenoyl or -SO-Z {wherein Z represents formula (A) :

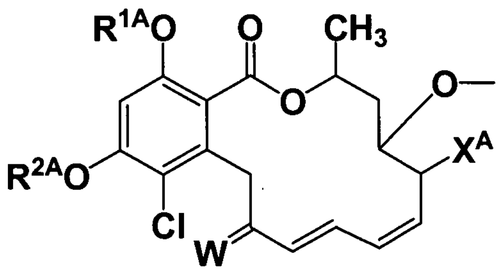

(A)

[wherein $R^{1A}$ and $R^{2A}$ have the same significances as the above $R^1$ and $R^2$, respectively, $X^A$ represents halogen, and W represents O or N-O-$R^{3A}$ (wherein $R^{3A}$ has the same significance as the above $R^3$)]}, or X and $R^4$ together represent a single bond]. The compounds represented by general formula (I) are hereinafter referred to as "Compounds (I)".

In the definitions of the groups in Compounds (I), unless otherwise specified, "lower" represents carbon numbers 1 to 8 and "higher" represents carbon numbers 9 to 30.

The alkanoyl includes straight-chain or branched alkanoyl groups having 1 to 30 carbon atoms, such as formyl, acetyl, propanoyl, isopropanoyl, butanoyl, caproyl, lauroyl, myristoyl, palmitoyl and stearoyl. The alkenoyl includes straight-chain or branched alkenoyl groups having 3 to 30 carbon atoms, such as acryloyl, methacryloyl, crotonoyl, isocrotonoyl, palmitoleoyl, linoleoyl and linolenoyl. The alkyl moiety of the lower alkyl and the lower alkoxy includes straight-chain or branched alkyl groups, such as methyl, ethyl, propyl, isopropyl, butyl, isobutyl, sec-butyl, tert-butyl, pentyl, isopentyl, neopentyl, hexyl, heptyl, octyl and isooctyl, and one of the carbon atoms thereof may be substituted by a silicon atom. The higher alkyl includes straight-chain or branched alkyl groups, such as decanyl, dodecyl and hexadecyl. The alkenyl includes straight-chain or branched alkenyl groups having 2 to 30 carbon atoms, such as vinyl, allyl, 1-propenyl, 2-butenyl, 1-pentenyl, 2-hexenyl, 1,3-pentadienyl, 1,3-hexadienyl, dodecenyl and hexadecenyl. The lower cycloalkyl includes those having 3 to 8 carbon atoms, such as cyclopropyl, cyclobutyl, cyclopentyl, cyclohexyl and cyclooctyl. The aryl includes phenyl, naphthyl, etc., and the aryl of the aroyl and the arylcarbamoyl has the same significance as this aryl. The heterocyclic group includes alicyclic heterocyclic groups and aromatic heterocyclic groups,

such as pyridonyl, pyrrolidonyl, uracilyl, dioxolanyl, pyrrolyl, tetrazolyl, pyrrolidinyl, thienyl, morpholino, thiomorpholino, piperazinyl, pyrazolidinyl, piperidino, pyridyl, homopiperazinyl, pyrazolyl, pyrazinyl, indolyl, isoindolyl, furyl, piperidyl, quinolyl, phthalazinyl, imidazolidinyl, imidazolinyl and pyrimidinyl. The heterocyclic group moiety of the carbonyl bonded to the heterocyclic group has the same significance as defined above, and examples of group names including the carbonyl are furoyl, tenoyl, nicotinoyl and isonicotinoyl. The substituted or unsubstituted heterocyclic groups formed by $R^6$ and $R^7$, and $R^{14}$ and $R^{15}$, respectively, together with the adjacent nitrogen atom (the heterocyclic group may further contain an oxygen atom, a sulfur atom or another nitrogen atom) include pyrrolidyl, morpholino, thiomorpholino, piperazinyl, pyrazolidinyl, pyrazolinyl, piperidino, homopiperazinyl, indolinyl, isoindolinyl, perhydroazepinyl, perhydroazocinyl, indolyl and isoindolyl. The alkylene includes groups formed by removing one hydrogen atom from the alkyl moiety of the above lower alkyl and higher alkyl. The halogen includes fluorine, chlorine, bromine and iodine atoms.

The substituted lower alkyl, the substituted higher alkyl, the substituted alkenyl, the substituted lower alkoxy and the substituted lower alkanoyl each have 1 to 3 substituents which are the same or different. Examples of the substituents include hydroxy, lower cycloalkyl, lower cycloalkenyl, lower alkoxy, lower alkanoyloxy, azido, amino, mono- or di-lower alkylamino, mono- or di-lower alkanoylamino, lower alkoxycarbonylamino, lower alkenyloxycarbonylamino, halogen, lower alkanoyl, substituted or unsubstituted aryl, a substituted or unsubstituted heterocyclic group, cyclic imido (which represents groups formed by removing the hydrogen atom bonded to the nitrogen atom of the imido), $CONR^{16}R^{17}$ [wherein $R^{16}$ and $R^{17}$, which may be the same or different, each represent a hydrogen atom, hydroxy, lower alkyl, lower cycloalkyl, higher alkyl, alkenyl, lower alkoxy, aryl, a heterocyclic group or $NR^{18}R^{19}$ (wherein $R^{18}$ and $R^{19}$, which may be the same or different, each represent hydrogen, lower alkyl, lower cycloalkyl, aryl, a heterocyclic group, lower alkanoyl, aroyl, carbonyl bonded to a heterocyclic group, or arylcarbamoyl)], $CO_2R^{20}$ (wherein $R^{20}$ represents a hydrogen atom, lower alkyl, higher alkyl, lower cycloalkyl, alkenyl, substituted or unsubstituted aryl, or a substituted or unsubstituted heterocyclic group) and $-(OCH_2CH_2)_nOCH_3$ (wherein n represents an integer of 1 to 10).

The substituted alkylene has 1 to 3 substituents which are the same or different. Examples of the substituents include hydroxy, lower alkoxy, lower alkanoyloxy, azido, amino, mono- or di-lower alkylamino, mono- or di-lower alkanoylamino, lower alkoxycarbonylamino, lower alkenyloxycarbonylamino, halogen, lower alkanoyl, substituted or unsubstituted aryl, substituted or unsubstituted pyridyl, substituted or unsubstituted pyridonyl, substituted or unsubstituted pyrrolidonyl, substituted or unsubstituted uracilyl, substituted or unsubstituted piperidyl, substituted or unsubstituted piperidino, substituted or unsubstituted pyrrolidinyl, substituted or unsubstituted morpholino, substituted or unsubstituted morpholinyl, substituted or unsubstituted piperazinyl, substituted or unsubstituted thiomorpholino, substituted or unsubstituted dioxolanyl, cyclic imido (which represents groups formed by removing the hydrogen atom bonded to the nitrogen atom of the imido), $CONR^{16}R^{17}$ (wherein $R^{16}$ and $R^{17}$ have the same significances as defined above) and $CO_2R^{20}$ (wherein $R^{20}$ has the same significance as defined above).

The substituted lower cycloalkyl, the substituted aryl, the substituted heterocyclic group, the substituted aroyl, the carbonyl bonded to a substituted heterocyclic group, the substituted arylcarbamoyl, the substituted pyridyl, the substituted pyridonyl, the substituted pyrrolidonyl, the substituted uracilyl, the substituted piperidyl, the substituted piperidino, the substituted pyrrolidinyl, the substituted morpholino, the substituted morpholinyl, the substituted piperazinyl, the substituted thiomorpholino, the substituted dioxolanyl, and the heterocyclic groups formed by $R^6$ and $R^7$, and $R^{14}$ and $R^{15}$, respectively, together with the adjacent nitrogen atom, each have 1 to 3 substituents which are the same or different. Examples of the substituents include hydroxy, lower alkyl or lower alkyl substituted by a heterocyclic group (the heterocyclic group may be substituted by lower alkyl), higher alkyl, alkenyl, lower cycloalkyl, lower cycloalkenyl, lower alkoxy, lower alkoxy lower alkoxy, lower alkanoyloxy, azido, amino, mono- or di-lower alkylamino, mono- or di-lower alkanoylamino, lower alkoxycarbonylamino, lower alkenyloxycarbonylamino, halogen, lower alkanoyl, aryl, a heterocyclic group, cyclic imido (which represents groups formed by removing the hydrogen atom bonded to the nitrogen atom of the imido), $CONR^{16}R^{17}$ (wherein $R^{16}$ and $R^{17}$ have the same significances as defined above), $CO_2R^{20}$ (wherein $R^{20}$ has the same significance as defined above) and $SO_2NR^{21}R^{22}$ (wherein $R^{21}$ and $R^{22}$, which may be the same or different, each represent a hydrogen atom or lower alkyl). Herein, the lower alkyl, the higher alkyl, the alkenyl, the lower cycloalkyl, the lower alkoxy, the halogen, the aryl, the aroyl, the arylcarbamoyl, the heterocyclic group and the carbonyl to which a heterocyclic group is bonded each have the same significance as defined above. The mono- or di-lower alkylamino, the lower alkoxycarbonyl and the lower alkoxycarbonylamino, and the lower alkyl of the lower alkoxy lower alkoxy each have the same significance as defined above. The lower alkenyl of the lower alkenyloxycarbonylamino includes the alkenyl groups having 2 to 8 carbon atoms among those mentioned in the above definition of the alkenyl, such as vinyl, allyl, 1-propenyl, 2-butenyl, 1-pentenyl, 2-hexenyl, 1,3-pentadienyl and 1,3-hexadienyl. The lower cycloalkenyl includes those having 4 to 8 carbon atoms, such as 2-cyclopentenyl, 2-cyclohexenyl and 1,3-cyclopentadienyl. The lower alkanoyl of the lower alkanoyl, the lower alkanoyloxy and the mono- or di-lower alkanoylamino includes the straight-chain or branched alkanoyl groups having 1 to 8 carbon atoms among those mentioned in the above definition of the alkanoyl, such as formyl, acetyl, propanoyl, isopropanoyl, butanoyl and caproyl. The cyclic imido includes phthalimido, succinimido, glutarimido, etc.

KF58333 is a compound represented by formula (B) below, i.e., a compound represented by general formula

(I) in which $R^1$ and $R^2$ each are a hydrogen atom, $R^3$ is 1-ethyl-2-pyrrolidonyl group, and X and $R^4$ together represent a single bond.

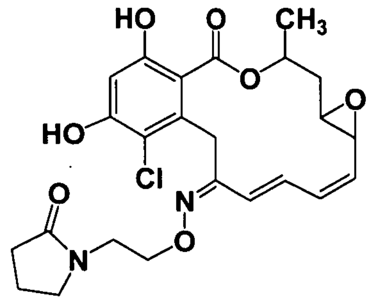

**(B)**

KF58333 and Compounds (I) can be produced by the method described in WO98/18780. The radicicol derivatives described in Japanese Published Unexamined Patent Application No. 226991/1992, WO96/33989 and WO99/55689 can be synthesized according to the methods described in Japanese Published Unexamined Patent Application No. 226991/1992, WO96/33989 and WO99/55689, respectively.

The derivatives of ansamycin compounds include 17-allylamino-17-demethoxy geldanamycin (17-AAG) and compounds described in US4261989, US5387584 and US5932566 which can be synthesized by the methods described in US4261989, US5387584 and US5932566. Further, 17-AAG is commercially available from Carbiochem. The purine derivatives binding to Hsp90 can be produced by the method described in WO02/36075.

The compounds having an inhibitory action on Hsp90 additionally include the compounds shown in the following (1) to (4).

(1) Cyclic phenylacetic acid derivatives represented by general formula (II):

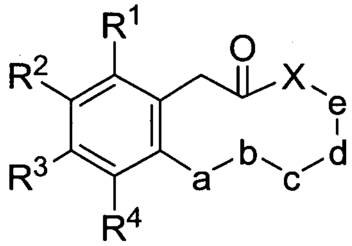

(II)

{wherein $R^1$, $R^2$, $R^3$ and $R^4$, which may be the same or different, each represent a hydrogen atom, hydroxy, substituted or unsubstituted lower alkyl, substituted or unsubstituted lower alkenyl, substituted or unsubstituted lower alkynyl, halogen, substituted or unsubstituted lower alkoxy, substituted or unsubstituted lower alkanoyloxy, substituted or unsubstituted aroyloxy, substituted or unsubstituted aralkyloxy, substituted or unsubstituted lower alkoxycarbonyloxy, carbamoyloxy, substituted or unsubstituted lower alkylaminocarbonyloxy, substituted or unsubstituted di-lower alkylaminocarbonyloxy, amino, substituted or unsubstituted lower alkylamino, substituted or unsubstituted di-lower alkylamino, substituted or unsubstituted lower alkanoylamino, substituted or unsubstituted lower alkylsulfonylamino, carbamoylamino, substituted or unsubstituted lower alkylcarbamoylamino, substituted or unsubstituted di-lower alkylcarbamoylamino, substituted or unsubstituted lower alkoxycarbonylamino, hydroxyamino, substituted or unsubstituted lower alkylthio, nitro, cyano, carboxy, substituted or unsubstituted lower alkoxycarbonyl, substituted or unsubstituted lower alkanoyl, substituted or unsubstituted aroyl, substituted or unsubstituted aryl, or substituted or unsubstituted aralkyl;
X represents an oxygen atom, a sulfur atom, -N($R^5$) (wherein $R^5$ represents a hydrogen atom or substituted or unsubstituted lower alkyl) or $-CH_2-$;
a represents $-CR^{a1}R^{a2}-$ [wherein $R^{a1}$ and $R^{a2}$, which may be the same or different, each represent a hydrogen atom, hydroxy, substituted or unsubstituted lower alkyl, substituted or unsubstituted lower alkoxy, substituted or unsubstituted aralkyloxy, substituted or unsubstituted aryloxy,
$-O(C{=}O)R^{a3}$ (wherein $R^{a3}$ represents substituted or unsubstituted lower alkyl, substituted or unsubstituted lower alkoxy,

amino, substituted or unsubstituted lower alkylamino, substituted or unsubstituted di-lower alkylamino, substituted or unsubstituted arylamino, or substituted or unsubstituted aralkylamino), substituted or unsubstituted aryl, or a substituted or unsubstituted heterocyclic group, or $R^{a1}$ and $R^{a2}$ together represent an oxygen atom (=O), =$NOR^{a4}$ (wherein $R^{a4}$ represents a hydrogen atom, substituted or unsubstituted lower alkyl, substituted or unsubstituted aryl, or substituted or unsubstituted aralkyl) or $-O(CH_2)_rO-$ (wherein r is 2 or 3)], substituted or unsubstituted arylene, a substituted or unsubstituted divalent heterocyclic group or a bond;

b represents $-(CR^{b1}R^{b2})_m-$ [wherein m represents an integer of 0 to 5, and $R^{b1}$ and $R^{b2}$, which may be the same or different, each represent a hydrogen atom, hydroxy, substituted or unsubstituted lower alkoxy, substituted or unsubstituted lower alkanoyloxy, or substituted or unsubstituted lower alkyl, or $R^{b1}$ and $R^{b2}$ together represent an oxygen atom (=O) ] or $-CHR^{b3}CR^{b4}R^{b5}-$ (wherein $R^{b3}$, $R^{b4}$ and $R^{b5}$, which may be the same or different, each represent a hydrogen atom, hydroxy, halogen, substituted or unsubstituted lower alkoxy, substituted or unsubstituted lower alkanoyloxy, amino, substituted or unsubstituted lower alkylamino, substituted or unsubstituted di-lower alkylamino, substituted or unsubstituted arylamino, substituted or unsubstituted lower alkylthio, substituted or unsubstituted arylthio, substituted or unsubstituted lower alkyl, substituted or unsubstituted aryl, or a substituted or unsubstituted heterocyclic group, or $R^{b3}$ and $R^{b4}$ together represent a bond or an oxygen atom, or $R^{b4}$ and $R^{b5}$ together represent an oxygen atom (=O));

c represents $-(CR^{c1}R^{c2})_n(CHR^{c3}CR^{c4}R^{c5})_p-$ (wherein n and p each have the same significance as the above m, $R^{c1}$ and $R^{c2}$ have the same significances as the above $R^{a1}$ and $R^{a2}$, respectively, and $R^{c3}$, $R^{c4}$ and $R^{c5}$ have the same significances as the above $R^{b3}$, $R^{b4}$ and $R^{b5}$, respectively); d represents $-(CR^{d1}CR^{d2})_q-$ (wherein q has the same significance as the above m, and $R^{d1}$ and $R^{d2}$ have the same significances as the above $R^{a1}$ and $R^{a2}$, respectively) or $-CHR^{d3}CR^{d4}R^{d5}-$ (wherein $R^{d3}$ , $R^{d4}$ and $R^{d5}$ have the same significances as the above $R^{b3}$, $R^{b4}$ and $R^{b5}$, respectively) ; and

e represents $-CR^{e1}R^{e2}-$ (wherein $R^{e1}$ and $R^{e2}$, which may be the same or different, each represent a hydrogen atom or substituted or unsubstituted lower alkyl)}

or pharmaceutically acceptable salts thereof.

The definitions of the groups in general formula (II) are explained below.

Examples of the lower alkyl moiety of the lower alkyl, the lower alkoxy, the lower alkoxycarbonyl, the lower alkylamino, the di-lower alkylamino, the lower alkylaminocarbonyloxy, the di-lower alkylaminocarbonyloxy, the lower alkylcarbamoylamino, the di-lower alkylcarbamoylamino, the lower alkoxycarbonylamino, the lower alkylsulfonylamino, the lower alkylthio and the lower alkoxycarbonyloxy include straight-chain or branched alkyl groups having 1 to 8 carbon atoms, such as methyl, ethyl, propyl, isopropyl, butyl, sec-butyl, tert-butyl, pentyl, isopentyl, neopentyl, hexyl, heptyl and octyl, and cycloalkyl groups having 3 to 8 carbon atoms, such as cyclopropyl, cyclobutyl, cyclopentyl, cyclohexyl, cycloheptyl and cyclooctyl. The two lower alkyl moieties of the di-lower alkylamino, the di-lower alkylaminocarbonyloxy and the di-lower alkylcarbamoylamino may be the same or different.

Examples of the lower alkenyl include straight-chain or branched alkenyl groups having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl groups having 2 to 8 carbon atoms, such as ethynyl, propynyl, butynyl, pentynyl, hexynyl, heptenyl and octynyl.

Examples of the lower alkanoyl moiety of the lower alkanoyl, the lower alkanoyloxy and the lower alkanoylamino include straight-chain or branched alkanoyl groups having 1 to 7 carbon atoms, such as formyl, acetyl, propionyl, butyryl, isobutyryl, valeryl, isovaleryl, pivaloyl, hexanoyl and heptanoyl.

Examples of the aryl moiety of the aryl, the aryloxy, the arylamino, the arylthio, the aroyl and the aroyloxy include aryl groups having 6 to 14 carbon atoms, such as phenyl, indenyl, naphthyl and anthryl.

Examples of the aralkyl moiety of the aralkyl, the aralkyloxy and the aralkylamino include aralkyl groups having 7 to 15 carbon atoms, such as benzyl, phenethyl, benzhydryl and naphthylmethyl.

The heterocyclic group includes aromatic heterocyclic groups and alicyclic heterocyclic groups. Examples of the aromatic heterocyclic groups include 5- or 6-membered monocyclic aromatic heterocyclic groups containing at least one atom selected from a nitrogen atom, an oxygen atom and a sulfur atom, and bicyclic or tricyclic condensed-ring aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl and purinyl. Examples of the alicyclic heterocyclic groups include 5- or 6-membered monocyclic alicyclic heterocyclic groups containing at least one atom selected from a nitrogen atom, an oxygen atom and a sulfur atom, and bicyclic or tricyclic condensed-ring alicyclic heterocyclic groups containing at least one atom selected from a nitrogen atom, an oxygen atom and a sulfur atom in which 3- to 8-membered rings are condensed, such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, tetrahydrofuranyl, tetrahydropyranyl and dihydrobenzofuranyl.

The halogen means fluorine, chlorine, bromine and iodine atoms.

The arylene represents groups in which one hydrogen atom is removed from the aryl defined above, and the divalent heterocyclic group represents groups in which one hydrogen atom is removed from the heterocyclic group defined above.

The substituted lower alkyl, the substituted lower alkenyl, the substituted lower alkynyl, the substituted lower alkoxy, the substituted lower alkanoyloxy, the substituted lower alkoxycarbonyloxy, the substituted lower alkylamino, the substituted di-lower alkylamino, the substituted lower alkanoylamino, the substituted lower alkanoyl, the substituted lower alkylaminocarbonyloxy, the substituted di-lower alkylaminocarbonyloxy, the substituted lower alkylsulfonylamino, the substituted lower alkylcarbamoylamino, the substituted di-lower alkylcarbamoylamino, the substituted lower alkoxycarbonylamino, the substituted lower alkylthio and the substituted lower alkoxycarbonyl each have 1 to 3 substituents which are the same or different. Examples of the substituents include hydroxy, cyano, carboxy and substituted or unsubstituted lower alkoxy. The positions to be substituted by the substituents are not specifically limited. The lower alkoxy has the same significance as defined above, and the substituted lower alkoxy has 1 to 3 substituents such as hydroxy.

The substituted aroyloxy, the substituted aralkyloxy, the substituted aryl, the substituted aroyl, the substituted aralkyl, the substituted aryloxy, the substituted arylamino, the substituted aralkylamino, the substituted arylthio, the substituted heterocyclic group, the substituted arylene and the substituted divalent heterocyclic group each have 1 to 3 substituents which are the same or different. Examples of the substituents include hydroxy, nitro, cyano, halogen, carboxy, lower alkoxycarbonyl, amino, lower alkylamino, di-lower alkylamino, lower alkanoyl, substituted or unsubstituted lower alkyl, and substituted or unsubstituted lower alkoxy. The positions to be substituted by the substituents are not specifically limited. The halogen, the lower alkoxycarbonyl, the lower alkylamino, the di-lower alkylamino, the lower alkanoyl, the lower alkyl and the lower alkoxy each have the same significance as defined above, and the substituted lower alkyl and the substituted lower alkoxy each have 1 to 3 substituents which are the same or different. Examples of the substituents include hydroxy and halogen, and the halogen has the same significance as defined above.

The compounds represented by general formula (II) are hereinafter referred to as "Compounds (II)".

<Production methods>

Compounds (II) can be produced, for example, by the methods described in literature [Japanese Published Unexamined Patent Application No. 287697/2000; Tetrahedron, 54, 15937-15958 (1998); Chin. Chem. Lett., 5, 481-484 (1994); Heterocycles, 32, 307-310 (1991); Tetrahedron Lett., 30, 2241-2244 (1989); Chem. Lett., 589-592 (1990); Nippon Kagaku Kaishi, 5, 883-885 (1981); Agrc. Biol. Chem., 40, 1663-1664 (1976); J. Chem. Soc. C., 10, 947-948 (1967), etc.] or methods similar thereto.

Further, a variety of Compounds (II) can be produced by converting functional groups according to known methods usually used in synthetic organic chemistry [e.g., R.C. Larock, Comprehensive Organic Transformations, second edition, John Wiley & Sons Inc. (1999)]. Also, the desired compounds having various functional groups can be produced by combining protection and deprotection of functional groups [e.g., T.W. Greene, Protective Groups in Organic Synthesis, John Wiley & Sons Inc. (1999)] according to need.

(2) Cyclic benzoic acid derivatives represented by general formula (III) :

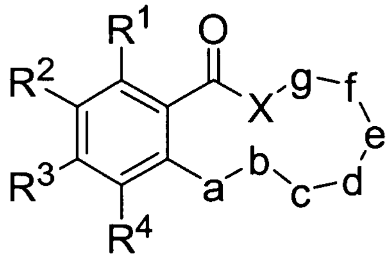

(III)

{wherein $R^1$, $R^2$, $R^3$ and $R^4$, which may be the same or different, each represent a hydrogen atom, hydroxy, substituted or unsubstituted lower alkyl, substituted or unsubstituted lower alkenyl, substituted or unsubstituted lower alkynyl, halogen, substituted or unsubstituted lower alkoxy, substituted or unsubstituted lower alkenyloxy, substituted or unsubstituted lower alkanoyloxy, substituted or unsubstituted aroyloxy, substituted or unsubstituted lower alkoxycarbonyloxy, carbamoyloxy, substituted or unsubstituted lower alkylaminocarbonyloxy, substituted or unsubstituted di-lower

alkylaminocarbonyloxy, amino, substituted or unsubstituted lower alkylamino, substituted or unsubstituted di-lower alkylamino, substituted or unsubstituted lower alkanoylamino, substituted or unsubstituted lower alkylsulfonylamino, carbamoylamino, substituted or unsubstituted lower alkylaminosulfonylamino, substituted or unsubstituted lower alkylaminocarbonylamino, nitro, cyano, carboxy, substituted or unsubstituted lower alkoxycarbonyl, substituted or unsubstituted lower alkanoyl, substituted or unsubstituted aryl, or substituted or unsubstituted aralkyl;
X represents an oxygen atom, a sulfur atom or -N($R^5$)-(wherein $R^5$ represents a hydrogen atom or substituted or unsubstituted lower alkyl);
a represents $-(CR^{a1}R^{a2})_m-$ (wherein m represents an integer of 0 to 5, and $R^{a1}$ and $R^{a2}$, which may be the same or different, each represent a hydrogen atom, hydroxy, substituted or unsubstituted lower alkoxy, substituted or unsubstituted lower alkanoyloxy, or substituted or unsubstituted lower alkyl, or $R^{a1}$ and $R^{a2}$ together represent an oxygen atom (=O)), $-CHR^{a3}CR^{a4}R^{a5}-$ (wherein $R^{a3}$, $R^{a4}$ and $R^{a5}$, which may be the same or different, each represent a hydrogen atom, hydroxy, substituted or unsubstituted lower alkoxy, substituted or unsubstituted lower alkanoyloxy, carboxy, substituted or unsubstituted lower alkoxycarbonyl, or substituted or unsubstituted lower alkylaminocarbonyl, or $R^{a3}$ and $R^{a4}$ together represent a bond or an oxygen atom, or $R^{a4}$ and $R^{a5}$ together represent an oxygen atom (=O)) or $-CHR^{a6}Y-$ (wherein $R^{6a}$ has the same significance as the above $R^{a3}$, and Y has the same significance as the above X);
b represents - $(CR^{b1}R^{b2})_n-$ (wherein n has the same significance as the above m, and $R^{b1}$ and $R^{b2}$ have the same significances as the above $R^{a1}$ and $R^{a2}$, respectively);
c represents $-(CHR^{c1}CHR^{c2})_p-$ (wherein p has the same significance as the above m, and $R^{c1}$ and $R^{c2}$, which may be the same or different, each represent a hydrogen atom, hydroxy, substituted or unsubstituted lower alkoxy, or substituted or unsubstituted lower alkanoyloxy, or $R^{c1}$ and $R^{c2}$ together represent a bond or an oxygen atom) or $-CHR^{c3}Z-$ (wherein $R^{c3}$ has the same significance as the above $R^{a1}$, and Z has the same significance as the above X) ;
d represents $-CR^{d1}R^{d2}-$ [wherein $R^{d1}$ and $R^{d2}$, which may be the same or different, each represent a hydrogen atom, hydroxy, halogen, cyano, amino, substituted or unsubstituted lower alkoxy, substituted or unsubstituted aralkyloxy, substituted or unsubstituted aryloxy, -O (C=O) $R^{d3}$ (wherein $R^{d3}$ represents substituted or unsubstituted lower alkyl, substituted or unsubstituted lower alkoxy, amino, substituted or unsubstituted lower alkylamino, substituted or unsubstituted di-lower alkylamino, or substituted or unsubstituted arylamino), substituted or unsubstituted lower alkylamino, substituted or unsubstituted di-lower alkylamino, substituted or unsubstituted arylamino, substituted or unsubstituted aralkylamino, substituted or unsubstituted lower alkanoylamino, substituted or unsubstituted lower alkyl, substituted or unsubstituted aryl, or a substituted or unsubstituted heterocyclic group, or $R^{d1}$ and $R^{d2}$ together represent an oxygen atom (=O), $=NOR^{d4}$ (wherein $R^{d4}$ represents a hydrogen atom, substituted or unsubstituted lower alkyl, substituted or unsubstituted aryl, or substituted or unsubstituted aralkyl) or $-O(CH_2)_rO-$(wherein r is 2 or 3)] or $-NR^{d4}(C=O)-$ (wherein $R^{d4}$ has the same significance as the above $R^5$);
e represents $-CR^{e1}R^{e2}-$ (wherein $R^{e1}$ and $R^{e2}$ have the same significances as the above $R^{a1}$ and $R^{a2}$, respectively) or $-CHR^{e3}CHR^{e4}-$ (wherein $R^{e3}$ and $R^{e4}$ have the same significances as the above $R^{a3}$ and $R^{a4}$, respectively) ;
f represents - $(CR^{f1}R^{f2})$ q- (wherein $R^{f1}$ and $R^{f2}$ have the same significances as the above $R^{a1}$ and $R^{a2}$, respectively, and q has the same significance as the above m); and
g represents $-CR^{g1}R^{g2}-$ (wherein $R^{g1}$ and $R^{g2}$, which may be the same or different, each represent a hydrogen atom or substituted or unsubstituted lower alkyl), provided that:

(i) when $R^1$ and $R^3$ each are hydroxy;
$R^2$ and $R^4$ each are a hydrogen atom;
X is an oxygen atom;
-a-b-c- is $-(CH_2)_4-$ or $-CH=CH-(CH_2)_2-$;
e and f each are $-CH_2-$; and
g is $-CH(CH_3)-$,
d is neither -C(=O)- nor -CH(OH)-, and
(ii) when $R^1$ is hydroxy or methoxy;
$R^3$ is hydroxy;
$R^2$ and $R^4$ each are a hydrogen atom;
X is an oxygen atom;
-a-b-c- is $-CH=CH- (CH_2)_2-$;
e-f- is $-CH_2-$; and
g is $-CH (CH_3)-$,
d is not -C(=O)-}
or pharmaceutically acceptable salts thereof.

The definitions of the groups in general formula (III) are explained below.

Examples of the lower alkyl moiety of the lower alkyl, the lower alkoxy, the lower alkoxycarbonyl, the lower

alkoxycarbonyloxy, the lower alkylamino, the di-lower alkylamino, the lower alkylaminocarbonyloxy, the di-lower alkylaminocarbonyloxy, the lower alkylsulfonylamino, the lower alkylaminosulfonylamino, the lower alkylaminocarbonylamino and the lower alkylaminocarbonyl include straight-chain or branched alkyl groups having 1 to 8 carbon atoms, such as methyl, ethyl, propyl, isopropyl, butyl, sec-butyl, tert-butyl, pentyl, isopentyl, neopentyl, hexyl, heptyl and octyl, and cycloalkyl groups having 3 to 8 carbon atoms, such as cyclopropyl, cyclobutyl, cyclopentyl, cyclohexyl, cycloheptyl and cyclooctyl. The two lower alkyl moieties of the di-lower alkylamino and the di-lower alkylaminocarbonyloxy may be the same or different.

Examples of the lower alkenyl moiety of the lower alkenyl and the lower alkenyloxy include straight-chain or branched alkenyl groups having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl groups having 2 to 8 carbon atoms, such as ethynyl, propynyl, butynyl, pentynyl, hexynyl, heptynyl and octynyl.

Examples of the lower alkanoyl moiety of the lower alkanoyl, the lower alkanoyloxy and the lower alkanoylamino include straight-chain or branched alkanoyl groups having 1 to 7 carbon atoms, such as formyl, acetyl, propionyl, butyryl, isobutyryl, valeryl, isovaleryl, pivaloyl, hexanoyl and heptanoyl.

Examples of the aryl moiety of the aryl, the aryloxy, the arylamino and the aroyloxy include aryl groups having 6 to 14 carbon atoms, such as phenyl, naphthyl and anthryl.

Examples of the aralkyl moiety of the aralkyl, the aralkyloxy and the aralkylamino include aralkyl groups having 7 to 15 carbon atoms, such as benzyl, phenethyl, benzhydryl and naphthylmethyl.

The heterocyclic group includes aromatic heterocyclic groups and alicyclic heterocyclic groups. Examples of the aromatic heterocyclic groups include 5- or 6-membered monocyclic aromatic heterocyclic groups containing at least one atom selected from a nitrogen atom, an oxygen atom and a sulfur atom, and bicyclic or tricyclic condensed-ring aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and phthalimido. Examples of the alicyclic heterocyclic groups include 5- or 6-membered monocyclic alicyclic heterocyclic groups containing at least one atom selected from a nitrogen atom, an oxygen atom and a sulfur atom, and bicyclic or tricyclic condensed-ring alicyclic heterocyclic groups containing at least one atom selected from a nitrogen atom, an oxygen atom and a sulfur atom in which 3- to 8-membered rings are condensed, such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, tetrahydrofuranyl, tetrahydropyranyl and dihydrobenzofuranyl.

The halogen means fluorine, chlorine, bromine and iodine atoms.

The substituted lower alkyl, the substituted lower alkoxy, the substituted lower alkenyl, the substituted lower alkynyl, the substituted lower alkenyloxy, the substituted lower alkanoyloxy, the substituted aroyloxy, the substituted lower alkoxycarbonyloxy, the substituted lower alkylaminocarbonyloxy, the substituted di-lower alkylaminocarbonyloxy, the substituted lower alkylamino, the substituted di-lower alkylamino, the substituted lower alkanoylamino, the substituted lower alkylsulfonylamino, the substituted lower alkylaminosulfonylamino, the substituted lower alkylaminocarbonylamino, the substituted lower alkoxycarbonyl, the substituted lower alkanoyl, the substituted aryl, the substituted heterocyclic group, the substituted aralkyl, the substituted lower alkylaminocarbonyl, the substituted aralkyloxy, the substituted arylamino and the substituted aralkylamino each have 1 to 3 substituents which are the same or different. Examples of the substituents include hydroxy, substituted or unsubstituted lower alkoxy, cyano, nitro, halogen, carboxy, formyl, amino, lower alkylamino, di-lower alkylamino, lower alkanoyl and lower alkoxycarbonyl. The positions to be substituted by the substituents are not specifically limited. The halogen, the lower alkoxy, the lower alkylamino, the di-lower alkylamino, the lower alkanoyl and the lower alkoxycarbonyl herein each have the same significance as defined above, and the substituted lower alkoxy has 1 to 3 substituents such as hydroxy.

The compounds represented by general formula (III) are hereinafter referred to as "Compounds (III)". The same applies to compounds of other formula numbers.

<Production methods>

Compounds (III) can be obtained as commercially available products or produced, for example, by the methods described in literature [US5795910; EP0606044; Chem. Lett., 172-173 (2001); Tetrahedron, 55, 8215-8230 (1999); J. Org. Chem., 43, 2339-2343 (1978); US3954805; US3925423; US3836544; US3810918; US3764614; US3373039; US3373030; US3239347; US3239342; US3239341, etc.] or methods similar thereto. Otherwise, they can be produced by the methods described in the literature or methods similar thereto from the commercially available products or compounds produced by the methods described in the literature or methods similar thereto.

Further, a variety of Compounds (III) can be produced by converting functional groups according to known

methods usually used in synthetic organic chemistry [e.g., R.C. Larock, Comprehensive Organic Transformations, second edition, John Wiley & Sons Inc. (1999)]. Also, the desired compounds having various functional groups can be produced by combining protection and deprotection of functional groups [e.g., T.W. Greene, Protective Groups in Organic Synthesis, John Wiley & Sons Inc. (1999)] according to need.

(3) Benzoyl compounds represented by general formula (IV):

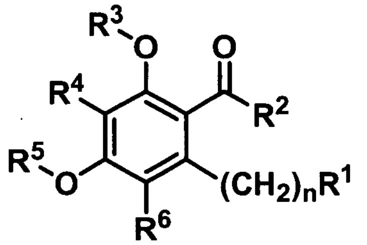

(IV)

[wherein n represents an integer of 0 to 10;
$R^1$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocyclic alkyl, substituted or unsubstituted aryl, substituted or unsubstituted arylsulfonyl, a substituted or unsubstituted heterocyclic group, $CONR^7R^8$ (wherein $R^7$ and $R^8$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocyclic alkyl, or substituted or unsubstituted aroyl, or $R^7$ and $R^8$ form a substituted or unsubstituted heterocyclic group together with the adjacent nitrogen atom) or $NR^9R^{10}$ (wherein $R^9$ and $R^{10}$ have the same significances as the above $R^7$ and $R^8$, respectively);
$R^2$ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group;
$R^3$ and $R^5$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl; and
$R^4$ and $R^6$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocyclic alkyl] ,
or pharmaceutically acceptable salts thereof.

The definitions of the groups in general formula (IV) are explained below.

Examples of the lower alkyl moiety of the lower alkyl, the lower alkoxy, the lower alkoxycarbonyl, the lower alkylamino and the di-lower alkylamino include straight-chain or branched alkyl groups having 1 to 8 carbon atoms, such as methyl, ethyl, propyl, isopropyl, butyl, sec-butyl, tert-butyl, pentyl, isopentyl, neopentyl, hexyl, heptyl and octyl. The two lower alkyl moieties of the di-lower alkylamino may be the same of different.

Examples of the lower alkenyl include straight-chain or branched alkenyl groups having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl groups having 2 to 8 carbon atoms, such as ethynyl, propynyl, butynyl, pentynyl, hexynyl, heptynyl and octynyl.

Examples of the lower alkanoyl moiety of the lower alkanoyl and the lower alkanoyloxy include straight-chain or branched alkanoyl groups having 1 to 7 carbon atoms, such as formyl, acetyl, propionyl, butyryl, isobutyryl, valeryl, isovaleryl, pivaloyl, hexanoyl and heptanoyl.

Examples of the cycloalkyl include cycloalkyl groups having 3 to 8 carbon atoms, such as cyclopropyl, cyclobutyl,

cyclopentyl, cyclohexyl, cycloheptyl and cyclooctyl.

Examples of the aryl moiety of the aryl, the arylsulfonyl, the aryloxy and the aroyl include monocyclic, bicyclic or tricyclic aryl groups having 6 to 14 carbon atoms, such as phenyl, indenyl, naphthyl and anthryl.

Examples of the aralkyl include aralkyl groups having 7 to 15 carbon atoms, such as benzyl, phenethyl, benzhydryl and naphthylmethyl.

The heterocyclic group moiety of the heterocyclic group and the heterocyclic alkyl includes aromatic heterocyclic groups and alicyclic heterocyclic groups. Examples of the aromatic heterocyclic groups include 5- or 6-membered monocyclic aromatic heterocyclic groups containing at least one atom selected from a nitrogen atom, an oxygen atom and a sulfur atom, and bicyclic or tricyclic condensed-ring aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and benzodioxolanyl. Examples of the alicyclic heterocyclic groups include 5- or 6-membered monocyclic alicyclic heterocyclic groups containing at least one atom selected from a nitrogen atom, an oxygen atom and a sulfur atom, and bicyclic or tricyclic condensed-ring alicyclic heterocyclic groups containing at least one atom selected from a nitrogen atom, an oxygen atom and a sulfur atom in which 3- to 8-membered rings are condensed, such as pyrrolidinyl, piperidino, piperazinyl, piperazinyl, morpholino, morpholinyl, thiomorpholino, thiomorpholinyl, homopiperidino, homopiperazinyl, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl and 2-oxopyrrolidinyl.

Examples of the heterocyclic group formed together with the adjacent nitrogen atom include 5- or 6-membered monocyclic heterocyclic groups containing at least one nitrogen atom (the monocyclic heterocyclic groups may also contain another nitrogen atom, oxygen atom or sulfur atom), and bicyclic or tricyclic condensed-ring heterocyclic groups containing at least one nitrogen atom in which 3-to 8-membered rings are condensed (the condensed-ring heterocyclic groups may also contain another nitrogen atom, oxygen atom or sulfur atom), such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, oxopiperazinyl and 2-oxopyrrolidinyl.

The alkylene moiety of the heterocyclic alkyl has the same significance as the above-described lower alkyl except one hydrogen atom is removed therefrom.

The halogen means fluorine, chlorine, bromine and iodine atoms.

The substituted lower alkyl, the substituted lower alkoxy, the substituted lower alkoxycarbonyl, the substituted lower alkenyl and the substituted lower alkynyl each have 1 to 3 substituents (A) which are the same or different. Examples of substituents (A) include hydroxy, oxo, cyano, nitro, carboxy, amino, halogen, substituted or unsubstituted lower alkoxy, cycloalkyl, lower alkanoyl, lower alkoxycarbonyl, lower alkylamino and di-lower alkylamino. The positions to be substituted by substituents (A) are not specifically limited. The halogen, the lower alkoxy, the cycloalkyl, the lower alkanoyl, the lower alkoxycarbonyl, the lower alkylamino and the di-lower alkylamino mentioned as examples of substituents (A) each have the same significance as defined above. The substituted lower alkoxy mentioned as an example of substituent (A) has 1 to 3 substituents which are the same or different, such as hydroxy and halogen, and the halogen has the same significance as defined above.

The substituted lower alkanoyl, the substituted lower alkanoyloxy, the substituted cycloalkyl, the substituted aryl, the substituted arylsulfonyl, the substituted aryloxy, the substituted aralkyl, the substituted aroyl, the substituted heterocyclic alkyl, the substituted heterocyclic group and the substituted heterocyclic group formed together with the adjacent nitrogen atom each have 1 to 3 substituents (B) which are the same or different. Examples of substituents (B) include hydroxy, halogen, nitro, cyano, amino, carboxy, carbamoyl, substituted or unsubstituted lower alkyl, substituted or unsubstituted lower alkoxy, aralkyloxy, lower alkylsulfonyl, lower alkylsulfanyl, cycloalkyl, lower alkoxycarbonyl, lower alkylamino, di-lower alkylamino, lower alkanoyl, a heterocyclic group, substituted or unsubstituted aryl, substituted or unsubstituted heterocyclic alkyloxy, and substituted or unsubstituted heterocyclic carbonylalkyloxy. The positions to be substituted by substituents (B) are not specifically limited. The halogen, the lower alkyl, the lower alkoxy, the cycloalkyl, the lower alkoxycarbonyl, the lower alkylamino, the di-lower alkylamino, the lower alkanoyl, the heterocyclic group and the aryl mentioned as examples of substituents (B) each have the same significance as defined above; the lower alkyl moiety of the lower alkylsulfonyl and the lower alkylsulfanyl has the same significance as the above-described lower alkyl; the aralkyl moiety of the aralkyloxy has the same significance as the above-described aralkyl; and the heterocyclic group moiety and the alkylene of the heterocyclic alkyloxy and the heterocyclic carbonylalkyloxy respectively have the same significance as the above-described heterocyclic group and the same significance as the above-described lower alkyl except one hydrogen atom is removed therefrom. The substituted lower alkyl, the substituted lower alkoxy and the substituted aryl mentioned as examples of substituents (B) each have 1 to 3 substituents which are the same or different, such as hydroxy, halogen, lower alkoxy, cyano, lower alkylamino and di-lower alkylamino. Herein, the halogen, the lower alkoxy, the lower alkylamino and the di-lower alkylamino each have the same significance as defined above. The substituted heterocyclic alkyloxy and the substituted heterocyclic carbonylalkyloxy mentioned as examples of substituents

(B) each have 1 to 3 substituents which are the same or different, such as hydroxy, halogen, lower alkyl, lower alkoxy and a heterocyclic group. Herein, the halogen, the lower alkyl, the lower alkoxy and the heterocyclic group each have the same significance as defined above.

Hereinafter, the compounds represented by general formula (IV) are referred to as "Compounds (IV)".

The processes for producing Compounds (IV) are described below.

In the processes shown below, when the defined groups undergo changes under the reaction conditions or are not suitable to carry out the processes, production can be easily performed by applying means usually used in synthetic organic chemistry, such as protection and deprotection of functional groups [e.g., T.W. Greene, Protective Groups in Organic Synthesis, John Wiley & Sons Inc. (1999)]. If necessary, the order of reaction steps such as introduction of a substituent may be changed.

<Production methods>

Compounds (IV) can be obtained, for example, according to Production Processes 1 to 6 shown below.

Production Process 1:

Compound (IV) can be produced, for example, according to the following step.

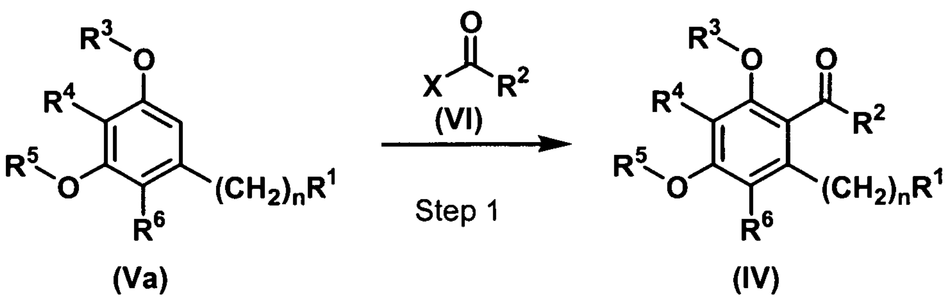


(In the formulae, $R^1$ to $R^6$ and n each have the same significance as defined above; and X represents hydroxy or halogen, wherein the halogen has the same significance as defined above.)

(Step 1)

Compound (IV) can be obtained by reacting Compound (Va) with 1 to 10 equivalents of Compound (VI) in an inert solvent in the presence of an acid.

Examples of the acid include organic acids such as acetic acid and trifluoroacetic acid, and Lewis acids such as aluminum trichloride and titanium tetrachloride. The acid is preferably used in an amount of 1 to 50 equivalents based on Compound (Va).

Examples of the inert solvent include dichloromethane and chloroform, but acetic acid, trifluoroacetic acid or the like may also be used as the solvent.

The reaction is usually carried out at a temperature between -50°C and the boiling point of the solvent used for 5 minutes to 24 hours. The reaction can be accelerated by adding 1 to 10 equivalents of acetic anhydride, trifluoroacetic anhydride or the like.

The starting Compound (Va) can be obtained according to a known method [e.g., R.C. Larock, Comprehensive Organic Transformations, second edition, John Wiley & Sons Inc. (1999)] or a method similar thereto.

Compound (VI) can be obtained as a commercially available product or according to a known method [e.g., R.C. Larock, Comprehensive Organic Transformations, second edition, John Wiley & Sons Inc. (1999)] or a method similar thereto.

It is possible to prepare Compound (Va-ii), i.e., Compound (Va) in which $R^6$ is $R^{6a}$ (wherein $R^{6a}$ represents substituted or unsubstituted lower alkanoyl, substituted or unsubstituted lower alkenyl, substituted or unsubstituted lower alkynyl, substituted or unsubstituted aryl, or a substituted or unsubstituted aromatic heterocyclic group in the definition of $R^6$), from Compound (Va-i), i.e., Compound (Va) in which $R^6$ is a hydrogen atom, according to a method similar to Production Process 6 described below.

It is also possible to obtain Compound (Va-iv), i.e., Compound (Va) in which $R^6$ is ethyl, by preparing Compound

(Va-iii), i.e., Compound (Va) in which $R^6$ is acetyl, from Compound (Va-i), i.e., Compound (Va) in which $R^6$ is a hydrogen atom, according to a method similar to the above Production Process 1, and then treating Compound (Va-iii) with triethylsilane or the like in trifluoroacetic acid or the like.

Production Process 2:

Compound (IV) can also be produced, for example, according to the following steps.

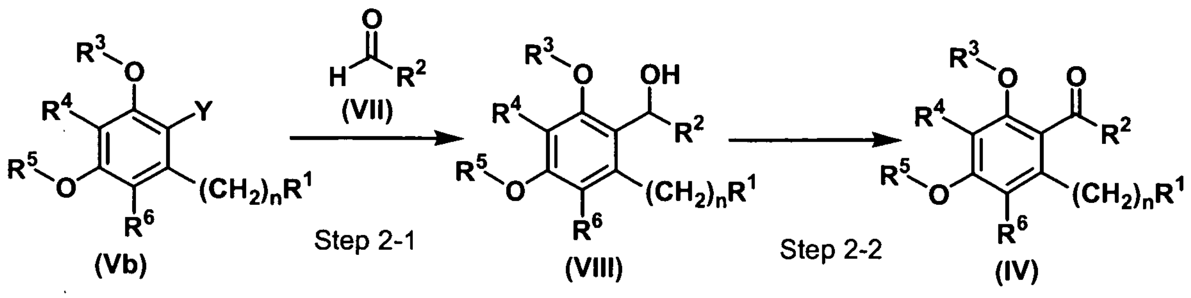


(In the formulae, $R^1$ to $R^6$ and n each have the same significance as defined above; and Y represents halogen, wherein the halogen has the same significance as defined above.)

(Step 2-1)

Compound (VIII) can be obtained by treating Compound (Vb) with 1 to 5 equivalents of a strong base such as n-butyllithium in an inert solvent and then reacting the resulting compound with Compound (VII).
Examples of the inert solvent include diethyl ether and tetrahydrofuran.
The reaction is usually carried out at a temperature between -78°C and 30°C for 5 minutes to 24 hours.
The starting Compound (Vb) can be obtained as a commercially available product or according to a known method [e.g., R.C. Larock, Comprehensive Organic Transformations, second edition, John Wiley & Sons Inc. (1999)] or a method similar thereto. Compound (Vb) can also be produced, for example, according to the following step.

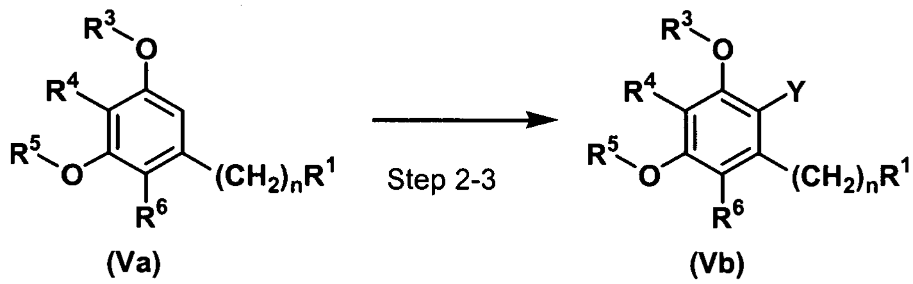


(In the formulae, $R^1$, $R^3$ to $R^6$, n and Y each have the same significance as defined above.)
Compound (Vb) can be obtained by treating Compound (Va) with 1 to 2 equivalents of a corresponding halogenating agent such as N-bromosuccinimide, N-chlorosuccinimide, chlorine, bromine or iodine in an inert solvent.
Examples of the inert solvent include dichloromethane, chloroform and N,N-dimethylformamide.
The reaction is usually carried out at a temperature between 0°C and 50°C for 5 minutes to 24 hours.

(Step 2-2)

Compound (IV) can be obtained by treating Compound (VIII) with 1 to 10 equivalents of an oxidizing agent in an inert solvent. Examples of the oxidizing agent include chromic acid, manganese dioxide, pyridinium dichromate (PDC) and 1-hydroxy-1,2-benziodoxol-3(1H)-one 1-oxide (IBX). This reaction may be carried out in the presence of molecular sieves.

Examples of the inert solvent include dichloromethane, chloroform, acetone, ethyl acetate and dimethyl sulfoxide.
The reaction is usually carried out at a temperature between 0°C and the boiling point of the solvent used for 5 minutes to 24 hours.

Production Process 3:

Compound (IVa), i.e., Compound (IV) in which $R^1$ is $CONR^7R^8$, can also be produced according to the following process.

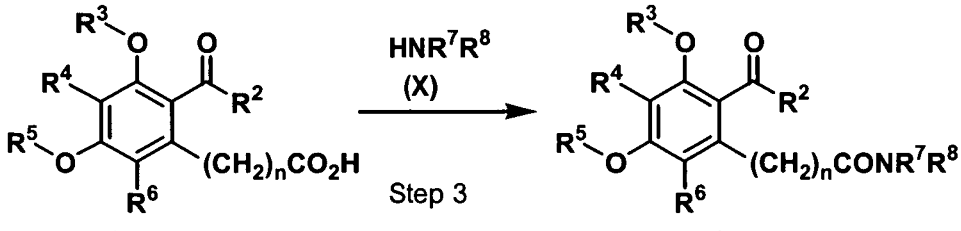


(IX) (IVa)

(In the formulae, $R^2$ to $R^8$ and n each have the same significance as defined above.)

(Step 3)

Compound (IVa) can be obtained by condensation reaction of Compound (IX) and Compound (X).
For example, Compound (IVa) can be obtained by reacting Compound (IX) with Compound (X) in a solvent in the presence of an activator such as 1-hydroxybenzotriazole or N-hydroxysuccinimide and a condensing agent. If necessary, 1 to 20 equivalents of a base may be added when the reaction is carried out. Usually, the condensing agent, the activator and Compound (X) are used in an amount of 1 to 20 equivalents based on Compound (IX), and the reaction is carried out at a temperature between -20°C and the boiling point of the solvent used for 1 minute to 24 hours.
Examples of the solvent include halogenated hydrocarbons such as dichloromethane and chloroform, esters such as methyl acetate, ethyl acetate and isobutyl acetate, ethers such as ether, tetrahydrofuran and 1,4-dioxane, aromatic hydrocarbons such as benzene and toluene, acetonitrile, N,N-dimethylformamide, N-methylpiperidone, and mixtures thereof.
Examples of the condensing agent include dicyclohexylcarbodiimide, 1-(3-dimethylaminopropyl)-3-ethylcarbodiimide hydrochloride, polymer-bound 1-(3-dimethylaminopropyl)-3-ethylcarbodiimide and triphenylphosphine oxide trifluoromethanesulfonic anhydride.
Examples of the base include alkylamines such as triethylamine, diisopropylethylamine and N-methylmorpholine, pyridines such as pyridine, lutidine, collidine and 4-dimethylaminopyridine, alkali metal carbonates such as potassium carbonate and sodium hydrogencarbonate, and alkali metal hydroxides such as potassium hydroxide, sodium hydroxide and lithium hydroxide.
Prior to use in the reaction, Compound (IX) may be treated with the activator, or the carboxyl group of Compound (IX) may be converted to a highly reactive group such as chlorocarbonyl, bromocarbonyl, p-nitrophenoxycarbonyl, pentafluorophenoxycarbonyl or pentafluorothiophenoxycarbonyl according to an ordinary method.
The starting Compound (IX) can be obtained according to Production Process 1, Production Process 2, a known method [e.g., J. Am. Chem. Soc., 93, 6708-6709 (1971)] or a method similar thereto. Compound (X) can be obtained as a commercially available product or according to a known method [e.g., R.C. Larock, Comprehensive Organic Transformations, second edition, John Wiley & Sons Inc. (1999)] or a method similar thereto.

Production Process 4:

Compound (IVc), i.e., Compound (IV) in which $R^3$ and $R^5$ each are a hydrogen atom, can also be produced from Compound (IVb), i.e., Compound (IV) in which $R^3$ is $R^{3a}$ (wherein $R^{3a}$ has the same significance as the above-described $R^3$ except a hydrogen atom is excluded) and $R^5$ is $R^{5a}$ (wherein $R^{5a}$ has the same significance as the above-described $R^5$ except a hydrogen atom is excluded), according to the following step.

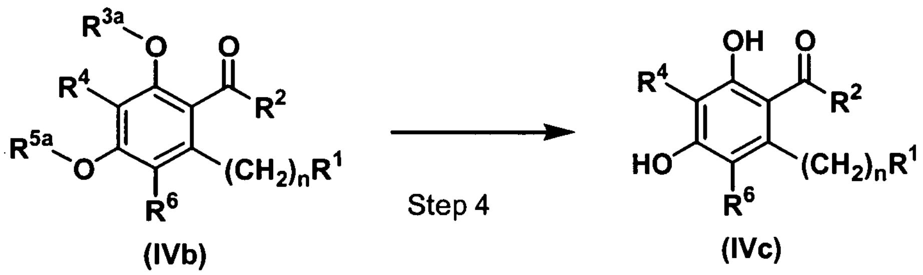


(In the formulae, $R^1$, $R^2$, $R^{3a}$, $R^4$, $R^{5a}$, $R^6$ and n each have the same significance as defined above.)

(Step 4)

Compound (IVc) can be obtained by treating Compound (IVb) with a Lewis acid such as boron tribromide, boron trichloride, boron trifluoride, aluminum trichloride, titanium tetrachloride or a complex thereof in an inert solvent such as dichloromethane. Usually, the Lewis acid is used in an amount of 1 to 20 equivalents based on Compound (IVb), and the reaction is carried out at a temperature between -78°C and the boiling point of the solvent used for 1 minute to 24 hours.

When the starting compound is Compound (IVb-i), i.e., Compound (IVb) in which $R^{3a}$ and $R^{5a}$ each are allyl, Compound (IVc) can also be obtained by treating Compound (IVb-i) with a nucleophilic agent, for example, a combination of a palladium complex such as bis(triphenylphosphine) palladium (II) dichloride and a formate such as ammonium formate, a typical metal hydride such as tributyltin hydride, a secondary amine such as morpholine, or an active methylene compound such as dimedone, in an inert solvent.

Examples of the inert solvent include tetrahydrofuran, acetic acid and 1,4-dioxane.

These reactions are usually carried out at a temperature between room temperature and the boiling point of the solvent used for 1 minute to 24 hours.

Compound (IVc) can also be obtained by treating Compound (IVb-i) with palladium (II) acetate in the presence or absence of a ligand such as triphenylphosphine or with a palladium complex such as tetrakis triphenylphosphine palladium (II), selenium dioxide or the like, in an organic acid such as acetic acid or formic acid or a mixed solvent of an organic acid and tetrahydrofuran.

These reactions are usually carried out at a temperature between room temperature and the boiling point of the solvent used for 1 minute to 24 hours.

When the starting compound is Compound (IVb-ii), i.e., Compound (IVb) in which $R^{3a}$ and $R^{5a}$ each are methoxymethyl, Compound (IVc) can also be obtained by treating Compound (IVb-ii) with an acid such as hydrochloric acid or acetic acid in a solvent.

Examples of the solvent include protic solvents such as water, methanol and isopropyl alcohol, and mixed solvents of a protic solvent and an inert solvent such as 1,4-dioxane.

These reactions are usually carried out at a temperature between 0°C and the boiling point of the solvent used for 5 minutes to 24 hours.

When $R^{3a}$ and $R^{5a}$ in Compound (IVb) are different from each other, the desired Compound (IVc) can be obtained by appropriately combining the above processes. Compound (IVd), i.e., Compound (IV) in which either $R^3$ or $R^5$ is a hydrogen atom, can be obtained from Compound (IVb) according to the above processes by adjusting the amount (equivalents) of the reagents used, the reaction temperature, and the like.

The starting Compound (IVb) can be obtained according to Production Process 1, Production Process 2, a known method [e.g., R.C. Larock, Comprehensive Organic Transformations, second edition, John Wiley & Sons Inc. (1999)] or a method similar thereto.

Production Process 5:

Compound (Va-v), i.e., Compound (Va) used as a starting compound in Production Process 1 or 2 in which $R^1$ is substituted or unsubstituted lower alkoxy, can also be produced according to the following process.

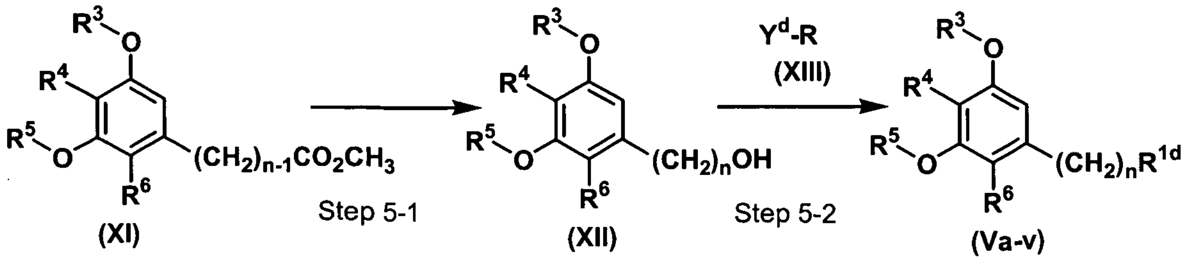


(XI) (XII) (Va-v)

(In the formulae, $R^3$ to $R^6$ and n each have the same significance as defined above; $Y^d$ has the same significance as the above-described Y; R represents substituted or unsubstituted lower alkyl; and $R^{1d}$ represents substituted or unsubstituted lower alkoxy, wherein the lower alkyl and the lower alkoxy each have the same significance as defined above, and the substituents in the substituted lower alkyl and the substituted lower alkoxy have the same significance as the above-described substituent in the substituted lower alkyl.)

(Step 5-1)

Compound (XII) can be obtained by treating Compound (XI) with 1 to 5 equivalents of a reducing agent such as isobutyl aluminum hydride or lithium aluminum hydride in an inert solvent.
Examples of the/inert solvent include tetrahydrofuran, toluene and dichloromethane.
The reaction is usually carried out at a temperature between -78°C and the boiling point of the solvent used for 5 minutes to 24 hours.
The starting Compound (XI) can be obtained according to Production Process 1, Production Process 2, a known method [e.g., R.C. Larock, Comprehensive Organic Transformations, second edition, John Wiley & Sons Inc. (1999)] or a method similar thereto.

(Step 5-2)

Compound (VA-v) can be obtained by treating Compound (XII) with 1 to 5 equivalents of sodium hydride or the like in an inert solvent and then reacting the resulting compound with 1 to 5 equivalents of Compound (XIII).
Examples of the inert solvent include tetrahydrofuran, dichloromethane and N,N-dimethylformamide.
The reaction is usually carried out at a temperature between 0°C and the boiling point of the solvent used for 5 minutes to 24 hours.

Production Process 6:

Compound (IVf), i.e., Compound (IV) in which $R^6$ is halogen, or Compound (IVg), i.e., Compound (IV) in which $R^6$ is $R^{6a}$ (wherein $R^{6a}$ has the same significance as defined above), can also be produced according to the following process.

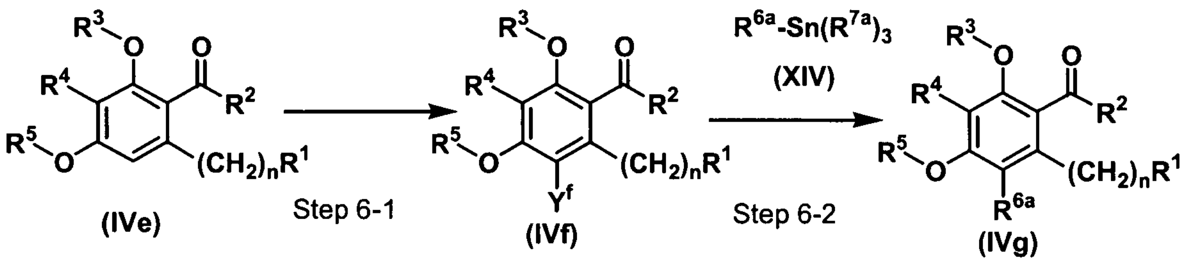


(IVe) (IVf) (IVg)

(In the formulae, $R^1$ to $R^5$, $R^{6a}$ and n each have the same significance as defined above; $Y^f$ has the same significance as the above-described Y; and $R^{7a}$ represents lower alkyl, wherein the lower alkyl has the same significance as defined above.)

(Step 6-1)

Compound (IVf) can be obtained by treating Compound (IVe) with 1 to 2 equivalents of a corresponding halogenating agent such as N-bromosuccinimide, N-chlorosuccinimide, chlorine, bromine or iodine in an inert solvent.
Examples of the inert solvent include dichloromethane, chloroform and N,N-dimethylformamide.
The reaction is usually carried out at a temperature between 0°C and 50°C for 5 minutes to 24 hours.
The starting Compound (IVe) can be obtained according to Production Processes 1 to 4, a known method [e.g., R.C. Larock, Comprehensive Organic Transformations, second edition, John Wiley & Sons Inc. (1999)] or a method similar thereto.

(Step 6-2)

Compound (IVg) can be obtained by reacting Compound (IVf) with 1 to 5 equivalents of Compound (XIV) in an inert solvent in the presence of 0.01 to 1 equivalent of bis(tri-o-tolylphosphine) palladium (II) dichloride, bis(triphenylphosphine) palladium (II) dichloride or the like and then, if necessary, treating the product with an acid such as hydrochloric acid.
Examples of the inert solvent include 1,2-dimethoxymethane, tetrahydrofuran, dichloromethane, chloroform, toluene and mixtures thereof.
The reaction is usually carried out at a temperature between 50°C and the boiling point of the solvent used for 5 minutes to 24 hours.
Compound (XIV) can be obtained as a commercially available product or according to a known method [e.g., R.C. Larock, Comprehensive Organic Transformations, second edition, John Wiley & Sons Inc. (1999)] or a method similar thereto.
In addition to the above-described production processes, Compounds (IV) can also be produced according to the methods described in WO01/81288; Japanese Published Unexamined Patent Application No. 92082/1996; Japanese Published Unexamined Patent Application No. 39968/2001; U.S. Patent No. 6,125,007; J. Antibiot., 55, 61-70 (2002); J. Am. Chem. Soc., 93, 6708-6709 (1971); Bioorg. & Med. Chem. Lett., 9, 1945-1948 (1999); Tetrahedron Lett., 43, 291-293 (2002); J. Chem. Soc. Perkin Trans. 1, 441-448 (1989); J. Chem. Soc. Perkin Trans. 1, 2502-2512 (1977); J. Chem. Soc. (C), 3899-3902 (1971); J. Chem. Soc. Perkin Trans. 1, 1417-1421 (1974); Tetrahedron Lett., 22, 267-270 (1981), etc., or methods similar thereto.
Further, the conversion of the functional groups in Compounds (IV), the starting compounds and the intermediates and the conversion.of the functional groups contained in the substituents can be carried out according to a known method [e.g., R.C. Larock, Comprehensive Organic Transformations, second edition, John Wiley & Sons Inc. (1999)] or a method similar thereto.
By appropriately combining the above-described processes and the like, Compounds (IV) having desired functional groups at desired positions can be obtained.
The intermediates and the desired compounds in the above-described production processes can be isolated and purified by appropriately combining separation and purification methods conventionally used in synthetic organic chemistry, for example, filtration, extraction, washing, drying, concentration, recrystallization, and various kinds of chromatography. The intermediates can also be subjected to the subsequent reactions without purification.
For some of the compounds having an inhibitory action on Hsp90 described above, there may exist stereoisomers such as geometrical isomers and optical isomers, and all possible isomers including them and mixtures thereof can be used for the therapeutic agent for diseases associated with immunoglobulin gene translocations of the present invention.
The pharmaceutically acceptable salts of compounds having an inhibitory action on Hsp90 include pharmaceutically acceptable acid addition salts, metal salts, ammonium salts, organic amine addition salts and amino acid addition salts.
Examples of the pharmaceutically acceptable acid addition salts of compounds having an inhibitory action on Hsp90 are inorganic acid addition salts such as hydrochloride, sulfate, nitrate and phosphate, and organic acid addition salts such as acetate, maleate, fumarate and citrate. Examples of the pharmaceutically acceptable metal salts are alkali metal salts such as sodium salt and potassium salt, alkaline earth metal salts such as magnesium salt and calcium salt, aluminum salt and zinc salt. Examples of the pharmaceutically acceptable ammonium salts are ammonium salt and tetramethylammonium salt. Examples of the pharmaceutically acceptable organic amine addition salts are salts with morpholine and piperidine. Examples of the pharmaceutically acceptable amino acid addition salts are salts with glycine, phenylalanine, lysine, aspartic acid and glutamic acid.
When it is desired to obtain a salt of a compound having an inhibitory action on Hsp90, in the case where the compound is produced in the form of the salt, it can be purified as such, but where the compound is produced in the free state, it can be converted into a salt by dissolving or suspending it in an appropriate solvent and then adding an

acid or a base thereto.

Further, the compounds having an inhibitory action on Hsp90 and pharmaceutically acceptable salts thereof may exist in the form of adducts with water or various solvents, and these adducts can also be used for the therapeutic agent for diseases associated with immunoglobulin gene translocations of the present invention.

3. Pharmaceutical preparations comprising a compound having an inhibitory action on Hsp90 or a pharmaceutically acceptable salt thereof as an active ingredient

The pharmaceutical preparations having an inhibitory action on Hsp90 of the present invention can comprise a compound having an inhibitory action on Hsp90 as the active ingredient alone or in combination with any other active ingredients for the therapy. These pharmaceutical preparations may be produced by any methods well known in the technical field of pharmaceutics by mixing the active ingredient with one or more pharmaceutically acceptable carriers.

It is desirable to select a route of administration that is most effective for the therapy, examples thereof being oral administration and intravenous and other parenteral administrations.

Examples of the dosage form include tablets, powders, granules, syrup and injections.

Liquid preparations suitable for oral administration such as syrup can be prepared using water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates) and flavors (e.g., strawberry flavor and peppermint). Tablets, powders, granules, etc. can be prepared using excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

Preparations suitable for parenteral administration preferably comprise a sterilized aqueous preparation containing an active compound which is isotonic to the recipient's blood. In the case of an injection, for example, a solution for injection is prepared using a carrier comprising a saline solution, a glucose solution, or a mixture of a saline solution and a glucose solution.

The parenteral preparations may also comprise one or more auxiliary components selected from the diluents, antiseptics, flavors, excipients, disintegrators, lubricants, binders, surfactants and plasticizers mentioned in the above description of oral preparations.

The dose and the administration schedule of the pharmaceutical preparation of the present invention will vary depending upon the administration route, the age and body weight of a patient, and the nature and degree of severeness of the symptom to be treated. Usually, in the case of oral administration, the active ingredient is administered in a dose of 0.01 mg to 1 g, preferably 0.05 to 50 mg, per adult once to several times per day. In the case of parenteral administration such as intravenous administration, the active ingredient is administered in a dose of 0.001 to 100 mg, preferably 0.01 to 10 mg, per adult once to several times per day. However, the dose and the administration schedule may vary depending upon various conditions as given above.

The compounds having an inhibitory action on Hsp90 and pharmaceutically acceptable salts thereof promote intracellular degradation of proteins encoded by the partner genes in immunoglobulin gene translocations and decrease them. That is, in diseases associated with immunoglobulin gene translocations, Hsp90 is considered to bind to proteins encoded by the translocation partner genes of which expression is abnormally enhanced, and thus maintain their stability. The compounds having an inhibitory action on Hsp90 and pharmaceutically acceptable salts thereof can be used as an agent for promoting intracellular degradation of the proteins encoded by the translocation partner genes.

In diseases associated with immunoglobulin gene translocations, development and progress of the diseases or their malignant progression is considered to be caused by abnormal enhancement of the expression of proteins encoded by the translocation partner genes. Pharmaceutical preparations comprising a compound having an inhibitory action on Hsp90 or a pharmaceutically acceptable salt thereof as an active ingredient inhibit development and progress of diseases associated with immunoglobulin gene translocations or their malignant progression by promoting intracellular degradation of the proteins and decreasing them. Therefore, they can be used as a therapeutic agent for such diseases.

In cells with immunoglobulin gene translocations, it is considered that cell growth is promoted because the expression of proteins encoded by the translocation partner genes is abnormally enhanced. The compounds having an inhibitory action on Hsp90 and pharmaceutically acceptable salts thereof can inhibit growth of the cells with immunoglobulin gene translocations by promoting intracellular degradation of the proteins and decreasing them.

Therapeutic effect may not be expected when a pharmaceutical preparation comprising a compound having an inhibitory action on Hsp90 or a pharmaceutically acceptable salt thereof as an active ingredient is administered to patients with diseases not associated with immunoglobulin gene translocations. However, sometimes, it is not possible to distinguish diseases associated with immunoglobulin gene translocations from those not associated with immunoglobulin gene translocations only by the symptoms, depending upon the diseases.

Whether or not a patient is with a disease associated with immunoglobulin gene translocations can be examined in the following manner based on the method described in literature [Blood, 94, 2583-2589 (1999)]: Cells are collected

from the diseased site of the patient. In the case of multiple myeloma, for example, plasma cells are collected from the bone marrow. After the collected cells are immobilized, fluorescence in situ hybridization (FISH) is carried out on the interphase nuclei of the cells using a probe specific to immunoglobulin gene and a probe specific to the partner gene in immunoglobulin gene translocation, which are each labeled with two kinds of fluorescent dyes having a different wavelength. When there is a translocation between immunoglobulin gene and the partner gene, spots where two kinds of fluorescent dyes adjoin are detected within the cells, whereas, when there is no translocation, the two kinds of fluorescent dyes do not adjoin but are detected separately. It is also possible to examine whether or not there is a translocation by isolating chromosomal DNA; carrying out PCR utilizing primers specific to immunoglobulin gene and the partner gene, respectively, using the chromosomal DNA as a template; and specifically detecting a fusion gene between immunoglobulin gene and the partner gene resulting from the translocation.

Thus, it is possible to effectively treat diseases associated with immunoglobulin gene translocations by administering a medicament comprising a compound having an inhibitory action on Hsp90 or a pharmaceutically acceptable salt thereof as an active ingredient to patients with such diseases.

## Brief Description of the Drawings

Fig. 1 shows disappearance of a protein encoded by the partner gene in immunoglobulin gene translocation within cells, that is, intracellular disappearance of FGFR3 when KF58333 is added to KMS-11 cells. The lanes show the results of Western blot analysis of KMS-11 cells to which KF58333 is not added or KF58333 is added to make the concentration of 4 nmol/l, 20 nmol/l and 100 nmol/l, respectively (from left to right), downwardly showing the results of detection of FGFR3, Raf1, Cdk6 and Erk-2, respectively.

Fig. 2 shows growth inhibition of KMS-11 cells by KF58333. The abscissa shows the concentration of KF58333 (nmol/l), and the ordinate shows the rate (%) of living cell count to that of control.

Fig. 3 shows antitumor effect of KF58333 on KMS-11 cell-transplanted mice. The abscissa shows days after the initiation of administration test, and the ordinate shows the ratio ($V/V_0$) of the volume of tumor (V) to that at day 0 of the administration test ($V_0$). ● shows the results in KF58333-administered mice and ○ shows those in control mice.

## Best Modes for Carrying Out the Invention

The present invention is described more in detail below referring to examples. These examples are not to be construed as limiting the scope of the invention.

## Example 1

Intracellular disappearance of a protein encoded by a partner gene in immunoglobulin gene translocation due to a compound having an inhibitory action on Hsp90

It was confirmed by Western blot carried out in the following manner that, in cells with immunoglobulin gene translocation, a protein encoded by the partner gene showing abnormal enhancement of expression due to the immunoglobulin gene translocation disappears within the cells by the addition of a compound having an inhibitory action on Hsp90.

KMS-11 cells derived from human multiple myeloma [In Vitro Cell. Dev. Biol., 25, 723 (1989)], which were diluted to 10,000 cells/ml with RPMI 1640 medium containing 10% fetal bovine serum (hereinafter referred to as "culturing medium"), were put in plastic culture flasks (125 $cm^2$, Nunc) in 30 ml portions. KMS-11 cells are reported to be the cells in which a large amount of FGFR3 is expressed as a result of translocation between immunoglobulin gene on 14q32 and FGFR3 gene on 4p16.3 [Blood, 90, 4062 (1997)]. A solution of radicicol derivative KF58333 [Blood, 96, 2284 (2000)], as the compound having an inhibitory action on Hsp90, in dimethyl sulfoxide (hereinafter abbreviated as "DMSO") prepared to have a concentration of 10 mmol/l was diluted to 1/10000 (concentration of KF58333: 1 $\mu$mol/l) with the culturing medium, and the resulting solution was added to the flasks so as to make the final concentration of 4.0 nmol/1, 20 nmol/l and 100 nmol/l, respectively. The KF85333 solution was not added to a control. After incubation was carried out in a 5% $CO_2$ incubator at 37°C for 40 hours, the cells were recovered by centrifugation at 1000G for 5 minutes.

To the thus recovered cells was added a cooled lysis buffer (50 mmol/l HEPES NaOH, pH 7.4, 250 mmol/l sodium chloride, 1 mmol/l ethylenediamine tetraacetate, 1% Nonidet P-40, 1 mmol/l dithiothreitol, 1 mmol/l phenylmethylsulfonyl fluoride, 5 $\mu$g/ml leupeptin), and the mixture was allowed to stand at 4°C for 30 minutes for cytolysis, followed by centrifugation at 20000G for 10 minutes. The concentration of proteins in the supernatant thus obtained was measured, and the samples were adjusted so that the amount of proteins per each lane was the same. Thereafter, separation of

proteins was carried out by SDS(sodium dodecyl sulfate)-polyacrylamide gel electrophoresis with 4 lanes.

The separated protein samples were each transferred to a polyvinylidene difluoride membrane (Millipore). Then, anti-FGFR3 antibody (Santa Cruz), anti-Raf-1 antibody (Santa Cruz), anti-Cdk6 antibody (Santa Cruz) and anti-Erk2 antibody (Upstate Biotechnology) were each added as primary antibodies and allowed to react with the proteins on the membrane, followed by further reaction with secondary antibodies, i.e., enzyme-labeled antibodies which react with respective primary antibodies [alkaline phosphatase-labeled anti-rabbit Ig antibody or anti-mouse Ig antibody (BIO-RAD)]. The band of each protein was detected by FluorImager (Molecular Dynamics) after reaction with ECF reagent (Molecular Dynamics).

As a result, as shown in Fig. 1, it was confirmed that not only Raf-1 and Cdk6 which were already reported to be Hsp90 client proteins but also FGFR3 encoded by the partner gene in immunoglobulin gene translocation disappeared by the addition of KF58333 having an inhibitory action on Hsp90. On the other hand, no change was observed in the amount of Erk2 which is not an Hsp90 client protein. Accordingly, it was found that a protein encoded by a partner gene in immunoglobulin gene translocation is an Hsp90 client protein and that the protein, like other Hsp90 client proteins, selectively disappears from the cells due to a compound having an inhibitory action on Hsp90.

Example 2

Growth inhibition of cells with immunoglobulin gene translocation by a compound having an inhibitory action on Hsp90

FGFR3 encoded by the partner gene in immunoglobulin gene translocation in KMS-11 cells is considered to participate in growth and malignant progression of tumors. Accordingly, the influence which a compound having an inhibitory action on Hsp90 exerts on growth of the cells by causing FGFR3 to disappear from the cells was examined.

KMS-11 cells diluted to $2 \times 10^5$ cells/ml with the culturing medium were pipetted into a 96-well microplate (Nunc) in an amount of 50 $\mu$l (1000 cells) per well. To each well was added 50 $\mu$l of a solution prepared by diluting a 10 mmol/l solution of KF58333 in DMSO to 600 nmol/l with the culturing medium and further serially diluting the resulting solution threefold with the culturing medium so as to make the final concentrations of KF58333 of 300, 100, 33.3, 11.1, 3.70 and 1.23 nmol/l, respectively. As a control, 50 $\mu$l of the culturing medium which does not contain KF58333 was added. Thereafter, incubation was carried out in a 5% $CO_2$ incubator at 37°C for 72 hours, and the number of living cells was measured in the following manner. After the completion of incubation, 20 $\mu$l of XTT [2,3-bis-(2-methoxy-4-nitro-5-sulfophenyl)-(2H)-tetrazolium-5-carboxyanilide] diluted twofold with the culturing medium was added to each well, followed by incubation in a 5% $CO_2$ incubator at 37°C for one hour. Then, the absorbance at 450 nm and 655 nm was measured using a microplate spectrophotometer (Model 550, BIO-RAD). A value (difference in absorbance) was calculated by subtracting the absorbance at 655 nm from that at 450 nm for each well. Since the number of living cells is proportional to the difference in absorbance, the rate (%) of the value of difference in absorbance obtained for cells to which various concentrations of KF58333 were added to that obtained for control cells to which KF58333 was not added when the latter value was designated as 100% was calculated as the rate of the number of living cells to that of control, and the cell growth inhibitory activity of KF58333 was measured.

As shown in Fig. 2, KF58333 showed cell growth inhibition against KMS-11 cells. Further, as a result of calculation from the graph of Fig. 2, the concentration of KF58333 to inhibit cell growth by 50% ($GI_{50}$ value) was 19.8 nmol/l. This concentration is almost equal to that of KF58333 causing the disappearance of gene products in Example 1, and it was suggested that the inhibition of cell growth is due to the disappearance of FGFR3.

From the foregoing, it was shown that a compound having an inhibitory action on Hsp90 inhibits growth of cells with immunoglobulin gene translocation and can be used as a therapeutic agent for diseases associated with immunoglobulin gene translocations, such as multiple myeloma.

Example 3

In vivo antitumor effect of a compound having an inhibitory action on Hsp90

In vivo antitumor effect of a compound having an inhibitory action on Hsp90 was examined using a human tumor model prepared by transplanting KMS-11 cells to immunodeficient mice as a model of a disease associated with immunoglobulin gene translocations.

KMS-11 cells were cultured in a 5% $CO_2$ incubator at 37°C using the culturing medium for propagation. One day before transplantation, 0.3 mg/mouse of anti-asialo GM1 antibody was intraperitoneally administered to 10 human multiple myeloma-derived KMS-11 cell Fox C.B-17/Icr-scidJcl mice (CLEA JAPAN). One day after the administration of the antibody, the cultured KMS-11 cells ($1 \times 10^7$ cells/mouse) were subcutaneously transplanted to the mice. Fourteen days after the transplantation, the longer diameter and shorter diameter of the tumor subcutaneously grown were measured with slide calipers, and the tumor volume was calculated according to the following equation:

$$\text{Tumor volume V (mm}^3\text{)} = \{\text{longer diameter (mm)} \times [\text{shorter diameter (mm)}]^2\}/2$$

At the same time, the body weight of each mouse was measured, and the mice were divided into two groups of 5 mice each so that the tumor volume and the body weight are randomized. The day was defined as day 0 of the administration test, and drug administration was started in the following manner.

To the KF58333 administration group, KF58333 dissolved in an administration solvent [a solution in which N, N-dimethylacetamide (Wako Pure Chemical Industries), CREMOPHOR EL (Sigma-Aldrich) and physiological saline (Otsuka Pharmaceutical) were mixed at a ratio by volume of 5:7.5:87.5] at a concentration of 2.5 mg/ml was intravenously administered from the caudal vein in a dose of 0.01 ml/g body weight of mouse (25 mg/kg) once a day for 5 consecutive days. To the non-drug administration control group, only the administration solvent was intravenously given in the same dose and for the same number of times as in the case of the KF58333 administration group. The tumor volume of the non-drug administration group and the KF58333 administration group was measured 4, 7, 10, 14, 17, 21 and 24 days after the initiation of the administration test, and the volume ratio ($V/V_0$) to the tumor volume at day 0 of the administration test ($V_0$) was calculated and compared. As a result, as shown in Fig. 3, apparent suppression of tumor growth was observed in the KF58333 administration group, and it was recognized that KF58333 has an antitumor effect.

From the foregoing, it was shown that diseases associated with immunoglobulin gene translocations such as multiple myeloma can be treated by the administration of a compound having an inhibitory action on Hsp90, and therefore, a medicament comprising a compound having an inhibitory action on Hsp90 or a pharmaceutically acceptable salt thereof as an active ingredient can be used ad a therapeutic agent for diseases associated with immunoglobulin gene translocations.

## Industrial Applicability

The present invention provides a therapeutic agent for diseases associated with immunoglobulin gene translocations such as multiple myeloma, a growth inhibitor of cells with immunoglobulin gene translocations, and an agent for promoting degradation of proteins encoded by partner genes in immunoglobulin gene translocations, which comprise a compound having an inhibitory action on Hsp90 or a pharmaceutically acceptable salt thereof as an active ingredient.

## Claims

1. A therapeutic agent for a disease associated with immunoglobulin gene translocations comprising, as an active ingredient, a compound having an inhibitory action on Hsp90 or a pharmaceutically acceptable salt thereof.

2. The therapeutic agent according to Claim 1, wherein the compound having an inhibitory action on Hsp90 has an inhibitory action on at least one Hsp90 among Hsp90α, Hsp90β, Grp94 and Hsp 75/TRAP1.

3. The therapeutic agent according to Claim 1, wherein the compound having an inhibitory action on Hsp90 is a compound binding to the ATP/ADP binding site of Hsp90.

4. The therapeutic agent according to Claim 3, wherein the compound binding to the ATP/ADP binding site of Hsp90 is radicicol or a derivative thereof represented by the following general formula (I):

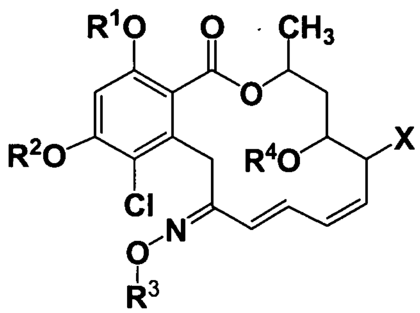

(I)

[wherein $R^1$ and $R^2$, which may be the same or different, each represent a hydrogen atom, alkanoyl, alkenoyl, tert-butyldiphenylsilyl or tert-butyldimethylsilyl,
$R^3$ represents Y-$R^5$ {wherein Y represents substituted or unsubstituted alkylene, and $R^5$ represents CONR$^6$R$^7$ [wherein $R^6$ represents a hydrogen atom, hydroxy, substituted or unsubstituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or NR$^8$R$^9$ (wherein $R^8$ and $R^9$, which may be the same or different, each represent a hydrogen atom, substituted or unsubstituted lower alkyl, substituted or unsubstituted higher alkyl, substituted or unsubstituted lower cycloalkyl, substituted or unsubstituted aryl, a substituted or unsubstituted heterocyclic group, substituted or unsubstituted alkanoyl, substituted or unsubstituted aroyl, carbonyl bonded to a substituted or unsubstituted heterocyclic group, or substituted or unsubstituted arylcarbamoyl), and $R^7$ represents hydroxy, substituted lower alkyl, substituted or unsubstituted higher alkyl, substituted or unsubstituted lower cycloalkyl, substituted or unsubstituted alkenyl, substituted or unsubstituted lower alkoxy, substituted or unsubstituted aryl, a substituted or unsubstituted heterocyclic group, or NR$^{10}$R$^{11}$ (wherein $R^{10}$ and $R^{11}$ have the same significances as the above $R^8$ and $R^9$, respectively), or $R^6$ and $R^7$ form a substituted or unsubstituted heterocyclic group together with the adjacent nitrogen atom], $CO_2R^{12}$ (wherein $R^{12}$ represents substituted lower alkyl, substituted or unsubstituted higher alkyl, substituted or unsubstituted lower cycloalkyl, substituted or unsubstituted alkenyl, substituted or unsubstituted aryl, or a substituted or unsubstituted heterocyclic group), substituted or unsubstituted aryl, substituted or unsubstituted pyridyl, substituted or unsubstituted pyridonyl, substituted or unsubstituted pyrrolidonyl, substituted or unsubstituted uracilyl, substituted or unsubstituted piperidyl, substituted or unsubstituted piperidino, substituted or unsubstituted pyrrolidinyl, substituted or unsubstituted morpholino, substituted or unsubstituted morpholinyl, substituted or unsubstituted piperazinyl, substituted or unsubstituted thiomorpholino, or substituted or unsubstituted dioxolanyl}, COR$^{13}$ [wherein $R^{13}$ represents a hydrogen atom, substituted or unsubstituted lower alkyl, substituted or unsubstituted higher alkyl, substituted or unsubstituted aryl, substituted or unsubstituted lower alkoxy, or NR$^{14}$R$^{15}$ (wherein $R^{14}$ and $R^{15}$, which may be the same or different, each represent a hydrogen atom, substituted or unsubstituted lower alkyl, substituted or unsubstituted higher alkyl, substituted or unsubstituted aryl, or substituted or unsubstituted pyridyl, or $R^{14}$ and $R^{15}$ form a substituted or unsubstituted heterocyclic group together with the adjacent nitrogen atom)] or substituted or unsubstituted aryl,
X represents halogen, and $R^4$ represents a hydrogen atom, alkanoyl, alkenoyl or -SO-Z {wherein Z represents formula (A) :

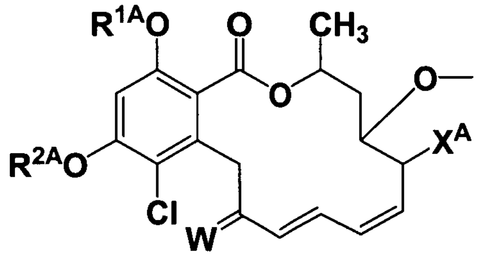

(A)

[wherein $R^{1A}$ and $R^{2A}$ have the same significances as the above $R^1$ and $R^2$, respectively, $X^A$ represents halogen, and W represents O or N-O-R$^{3A}$ (wherein $R^{3A}$ has the same significance as the above $R^3$)]}, or X and $R^4$ together represent a single bond].

5. The therapeutic agent according to any one of Claims 1 to 4, wherein the immunoglobulin gene translocation is at the q32 region on chromosome 14 (14q32).

6. The therapeutic agent according to Claim 5, wherein the translocation at 14q32 is t(11;14) (q13;q32), t (6; 14) (p21; q32), t (4;14) (p16;q32), t (14;16) (q32;q23), t (6;14) (p25;q32), t(1;14) (q21-24;q32), t(14;20) (q32;q12), t(1;14) (q10-12; q32), t (2; 14) (p13; q32), t(3; 14) (q21; q32), t(4; 14) (q22-33; q32), t (9; 14) (p13; q32), t (11; 14) (q23; q32), t (12; 14) (p13;q32), t(14;21)(q32;q22) or t (14;22) (q32;q12) .

7. The therapeutic agent according to Claim 5, wherein the partner gene in the translocation at 14q32 is cyclin D1 gene on 11q13, cyclin D3 gene on 6p21, FGFR3 gene or MMSET gene on 4p16, c-maf gene on 16q23, MUM1/IRF-4 gene on 6p25, IRTA1 gene or IRTA2 gene on 1q21-24, or mafB gene on 20q12.

**8.** The therapeutic agent according to any one of Claims 1 to 7, wherein the disease associated with immunoglobulin gene translocations is a hematopoietic tumor.

**9.** The therapeutic agent according to Claim 8, wherein the hematopoietic tumor is leukemia, multiple myeloma or lymphoma.

**10.** A growth inhibitor for a cell with immunoglobulin gene translocation comprising, as an active ingredient, a compound having an inhibitory action on Hsp90 or a pharmaceutically acceptable salt thereof.

**11.** An agent for promoting degradation of a protein encoded by a partner gene in immunoglobulin gene translocation comprising, as an active ingredient, a compound having an inhibitory action on Hsp90 or a pharmaceutically acceptable salt thereof.

**12.** Use of a compound having an inhibitory action on Hsp90 or a pharmaceutically acceptable salt thereof for the manufacture of a pharmaceutical composition for the treatment of a disease associated with immunoglobulin gene translocations.

**13.** A method of screening for a patient, which comprises: collecting cells from patients; examining the presence or absence of immunoglobulin gene translocation on the chromosome of the cells; and selecting a patient with immunoglobulin gene translocation as a target patient to administer a medicament comprising, as an active ingredient, a compound having an inhibitory action on Hsp90 or a pharmaceutically acceptable salt thereof as a therapeutic agent.

**14.** The method according to Claim 13, wherein the patient is a hematopoietic tumor patient.

**15.** The method according to Claim 14, wherein the hematopoietic tumor is leukemia, multiple myeloma or lymphoma.

**16.** A method of treating a disease associated with immunoglobulin gene translocations, which comprises: collecting cells from patients; examining the presence or absence of immunoglobulin gene translocation on the chromosome of the cells; and administering a medicament comprising, as an active ingredient, a compound having an inhibitory action on Hsp90 or a pharmaceutically acceptable salt thereof to a patient with immunoglobulin gene translocation.

**INTERNATIONAL SEARCH REPORT**

International application No.
PCT/JP2004/012418

A. CLASSIFICATION OF SUBJECT MATTER
Int.Cl$^7$ A61K45/00, A61K31/365, A61P35/00, A61P35/02, A61P43/00//
C07D313/00, C07D493/04

According to International Patent Classification (IPC) or to both national classification and IPC

B. FIELDS SEARCHED

Minimum documentation searched (classification system followed by classification symbols)
Int.Cl$^7$ A61K45/00, A61K31/365, A61P35/00, A61P35/02, A61P43/00,
C07D313/00, C07D493/04

Documentation searched other than minimum documentation to the extent that such documents are included in the fields searched

Electronic data base consulted during the international search (name of data base and, where practicable, search terms used)
CAP(STN), BIOSIS(STN), MEDLINE(STN), EMBASE(STN), WPIDS(STN), JOIS

C. DOCUMENTS CONSIDERED TO BE RELEVANT

| Category* | Citation of document, with indication, where appropriate, of the relevant passages | Relevant to claim No. |
|---|---|---|
| X | SHIOTSU Y. et al., 'Novel oxime derivative of radicicol induce erthroid differentiation associated with preferential G(1) phase accumulation against chromic myelogenous leukemia cells through destabilization of Bcr-Abl with Hsp90 complex.', Blood, (2000), Vol.96, No.6, pages 2284 to 2291; full text; registry no.308244-21-5 | 1-12 |
| X | SHIOTSU Y. et al., 'Heat shock protein 90-antagonist destabilizes Bcr-Abl/HSP chaperone complex.', Leukemia and Lymphoma, (2002), Vol.43, No.5, pages 961 to 968; full text; registry no.308244-21-5 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 November, 2004 (23.11.04) | 14 December, 2004 (14.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/012418

C (Continuation). DOCUMENTS CONSIDERED TO BE RELEVANT

| Category* | Citation of document, with indication, where appropriate, of the relevant passages | Relevant to claim No. |
|---|---|---|
| X | WO 02/69900 A2 (CONFORMA THERAPEUTICS CORP.), 12 September, 2002 (12.09.02), Full text; Claims 1, 6, 12, 13, 24, 27, 36, 37 & AU 2002/252179 B & EP 1423080 A2 | 1-12 |
| X | GORRE ME. et al., 'BCR-ABL point mutants isolated patients with imatinib mesylate-resistant chronic myeloid leukemia remain sensitive to inhibitors of the BCR-ABL chaperone heat shock protein 90.', Blood (2002), Vol.100, No.8, pages 3041 to 3044; full text | 1-3,5-12 |
| X | Chem.Abstr. (2001), Vol.137, abstract no. 27882 & NAOE T. et al., 'FLT3 tyrosine kinase as a target molecule for selective antileukemia therapy.", Cancer Chemotherapy and Pharmacology, (2001), Vol.48, no.suppl.1, p.S27-S30 | 1-3,5-12 |
| X | BLAGOSKLONNY MV. et al., 'The Hsp90 inhibitor geldanamycin selectively sensitizes Bcr-Abl-expressing leukemia cells to cytotoxic chemotherapy.', Leukemia, (2001), Vol.15, no.10, pages 1537 to 1543; full text | 1-3,5-12 |
| X | DATABASE BIOSIS, (ENTERED STN: 06 August, 2003 (06.08.03)), abstract no. 2003:356443 abstract & MITSIADES CS et al., 'Hsp90 inhibitors prolong survival in a SCID/NOD mice model of diffuse multiple myeloma: therapeutic implications.', Blood, (2002), Vol.100, No.11, abstract no.392 | 1-3,5-12 |
| X | DATABASE BIOSIS, (ENTERED STN: 23 July, 2003 (23.07.03)) abstract no.2003:336889 abstract & YAO Q. et al., 'FLT3 expressing MLL fusion gene leukemias are selectively sensitive to inhibitors of the molecular chaperone hsp90 through destabilization of signal-transduction -associated kinases.', Blood (2002) Vol.100, No.11, abstract no.2192 | 1-3,5-12 |
| X | DATABASE BIOSIS(ENTRED STN: 13 May, 2002 (13.05.02)) abstract no. 2002:186727 abstract & MITSIADES CS et al., 'The hsp90 molecular chaperon as a novel therapeutic target in hematologic malignancies.', Blood (2001), Vol.98, No.11, part 1 abstract no.377a | 1-3,5-12 |

Form PCT/ISA/210 (continuation of second sheet) (January 2004)

**INTERNATIONAL SEARCH REPORT**

| International application No. |
|---|
| PCT/JP2004/012418 |

C (Continuation). DOCUMENTS CONSIDERED TO BE RELEVANT

| Category* | Citation of document, with indication, where appropriate, of the relevant passages | Relevant to claim No. |
|---|---|---|
| X | DATABASE BIOSIS, (ENTERED STN: 06 February, 2002 (06.02.06)), abstract no. 2002:129931 abstract & MINAMI Y. et al., 'Selective apoptosis of mutant FLT3-transformed leukemia cells by hsp90-inhibitors.', Blood (2001), Vol.98, No.11, part 1, abstract no.377a | 1-3,5-12 |
| X | DATABASE BIOSIS, (ENTERED STN: 07 November, 2001 (07.11.01)), abstract no.2001:521611 abstract & BONVINI P. et l., '17-allylaminoa -17-demethoxygeldanamycin (17-AAG) decreases NPM-ALK steady-state and perturbs NPM-ALK dependent tyrosine phosphorylation by inhibiting NPM-ALK/hsp90 complex.', British Journal of Cancer (2001), Vol.85, No.suppl.1, p.91 | 1-3,5-12 |
| A | FINELLI PF. et al., 'Detection of t(4;14) (p16. 3;q32) chromosomal translocation in multiple myeloma by double-color fluorescent in situ hybridization.', Blood (1999), Vol.94, No.2, pages 724 to 732, full text | 1-12 |
| A | RONCHETTI D. et al., 'Deregulated FGFR3 mutants in multiple myeloma cell lines with t(4;14): comparative analysis of Y373C, K650E and the novel G384D mutations.' Oncogene, (2001), Vol.20, No.27, pages 3553 to 3562; full text | 1-12 |
| A | RICHELDA R. et al., 'A novel chromosomal translocation t(4;14) (p16.3;q32) in multiple myeloma involves the fibroblast growth-factor receptor 3 gene.', Blood, (1997), Vol.90, No.10, pages 4062 to 4070; full text | 1-12 |
| A | DATABASE BIOSIS, (ENTERED STN: 20 July, 2001 (20.07.01)), abstract no. 2001:299266 abstract & FONSECA R. et al., 'Dysregulation of c-MAF is associated with t(14;16) (q32;q23), breakpoints that flank the fra 16D fragile site within the WWOX gene, and can be greater than 1 Mb from-c-maf.', Blood, (2000), Vol.96, No.11, part 2, p.158b | 1-12 |

Form PCT/ISA/210 (continuation of second sheet) (January 2004)

**INTERNATIONAL SEARCH REPORT**

International application No.
PCT/JP2004/012418

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 13-16
because they relate to subject matter not required to be searched by this Authority, namely:
Claims 13 to 15 involve embodiments concerning methods for treatment of the human body by diagnostic methods and claim 16 involves embodiments concerning methods for treatment of the human body by therapy and thus relate to a subject matter (continued to extra sheet.)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.
PCT/JP2004/012418

Continuation of Box No.II-1 of continuation of first sheet(2)

which this International Searching Authority is not required, under the provisions of Article 17(2)(a)(i) of the PCT and Rule 39.1(iv) of the Regulations under the PCT, to search.

<Scope of search>

Claim 1 relates to a remedy for "diseases associated with the translocation of immunoglobulin gene" which contains, as the active ingredient, a compound defined by a desired property "having an Hsp90 inhibitory effect". Although claim 1 involves any compounds having such a property, it is recognized that only small part of the claimed compounds are supported by the description in the meaning within PCT Article 6 and disclosed therein in the meaning within PCT Article 5.

Although the common technical knowledge at the point of the application is taken into consideration, the scope of the compound having the property as "a compound having an Hsp90 inhibitory effect" cannot be specified. Thus, claim 1 does not comply with the requirement of clearness in the meaning within PCT Article 6 too.

Such being the case, the search was made mainly on the relationship between the Hsp90 inhibitory effect and "diseases associated with the translocation of immunoglobulin gene" or hematopoietic organ tumors typified by leukemia, multiple myeloma and lymphoma and remedies for hematopoietic organ tumors which contain, as the active ingredient, radicicol or its derivatives such as K58333 that are defined in claim 4 and employed in the specification in practice.